(19) **Europäisches Patentamt European Patent Office Office européen des brevets**

(11) **EP 2 789 400 B1**

(12) **EUROPEAN PATENT SPECIFICATION**

(45) Date of publication and mention of the grant of the patent:
**19.07.2017 Bulletin 2017/29**

(51) Int Cl.:
*B05C 19/06* (2006.01)  *B05D 3/00* (2006.01)
*B65G 27/32* (2006.01)  *B65G 65/46* (2006.01)
*G01G 13/20* (2006.01)  *A61F 7/03* (2006.01)
*H01M 10/00* (2006.01)  *A61F 7/02* (2006.01)
*C09K 5/16* (2006.01)  *B05C 19/04* (2006.01)
*B05C 11/10* (2006.01)  *B05D 1/30* (2006.01)
*G01G 11/08* (2006.01)  *C09K 5/18* (2006.01)
*A61F 7/00* (2006.01)

(21) Application number: **12856305.3**

(22) Date of filing: **30.11.2012**

(86) International application number:
**PCT/JP2012/081198**

(87) International publication number:
**WO 2013/084831 (13.06.2013 Gazette 2013/24)**

(54) **METHOD AND DEVICE FOR APPLYING POWDER OR GRANULE AND METHOD FOR MANUFACTURING A HEAT GENERATING ELEMENT USING SAID METHOD**

VERFAHREN UND VORRICHTUNG ZUM AUFTRAGEN VON PULVER ODER GRANULAT UND VERFAHREN ZUM HERSTELLEN EINES WÄRMEERZEUGENDEN ELEMENTS DAMIT

PROCÉDÉ ET DISPOSITIF D'APPLICATION D'UNE POUDRE  ET PROCÉDÉ D'APPLICATION POUR LA FABRICATION D'UN ÉLÉMENT CHAUFFANT À L'AIDE DE CE PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.12.2011 JP 2011268413**

(43) Date of publication of application:
**15.10.2014 Bulletin 2014/42**

(73) Proprietor: **Kao Corporation Chuo-Ku Tokyo 103-8210 (JP)**

(72) Inventors:
• **OKU, Shogo
  Haga-gun
  Tochigi 321-3497 (JP)**
• **SUGAI, Satoru
  Haga-gun
  Tochigi 321-3497 (JP)**
• **OOTSUKA, Kazutoshi
  Haga-gun
  Tochigi 321-3497 (JP)**

(74) Representative: **Vossius & Partner Patentanwälte Rechtsanwälte mbB Siebertstrasse 3 81675 München (DE)**

(56) References cited:
WO-A1-2007/081014    DE-C1- 19 517 070
JP-A- H 095 148        JP-A- H1 099 367
JP-A- S62 174 617      JP-A- 2001 253 534
JP-A- 2006 320 796

## Description

FIELD OF THE INVENTION

The present invention relates to a method and a device for applying (sprinkling or disseminating) a powder or granule, particularly, a powder or granule which has highly deliquescent or hygroscopic properties, and a method for manufacturing a heat generating element using the same.

BACKGROUND OF THE INVENTION

Various works have been disclosed regarding methods for quantitatively supplying a powder or granule onto a base material to be continuously conveyed, and a device therefor. For example, JP-A-04-341368 discloses a transfer-type sprinkle device of a powder or granule, which sprinkles the powder or granule intermittently by vacuuming part of a continuous flow of the powder or granule supplied from a powder or granule supplier (screw feeder).

Moreover, JP-A-11-153473discloses a quantitative supply-delivery device for a powder or granule, which has a supply device, a delivery device comprising a screw feeder, a joint means and a measuring device. The joint means is located upstream of the screw feeder and configured to be vibrated by a vibrator. Moreover, the joint means is separated from an outlet of the supply device. Thus, it is said to enable prevention of solidification of the powder or granule supplied to the screw feeder. Further, vibration of the vibrator or the screw feeder is not readily transmitted from the joint means to the supply device, and the measuring device is said to enable suitable measurement of a decrement of the powder or granule in the supply device, thereby ensuring accurate supply of the powder or granule.

JP H10 99367 A discloses another supply device for powder having a screw feeder, a vibration feeder, and a screw conveyor.

However, these conventional devices give no particular attention to possible supply of a powder or granule which is highly deliquescent or hygroscopic and coagulable, or a powder or granule having a property of easily forming lumps even under slight pressure, or to prevention thereof.

SUMMARY OF THE INVENTION

The invention is defined in the claims. The present invention provides a method for applying a powder or granule on a base material to be continuously conveyed, wherein the powder or granule is continuously pulled out, by a screw feeder, from a supply unit for temporarily storing the powder or granule, the pulled-out powder or granule is allowed to fall to be received in a vibrating conveyor unit, the powder or granule is conveyed while being dispersed by vibration of a vibrating element of the vibrating conveyor unit, and the powder or granule is continuously applied on the base material from a sprinkling outlet of the vibrating conveyor unit; characterized in that a first measuring means continuously measures the total mass of the supply unit, the screw feeder and the powder or granule therein, and a screw control unit calculates the decrement of the powder or granule per time based on a plurality of the measured values, and controls the screw rotation speed of the screw feeder so that the calculated decrement coincides with a target discharge amount per time.

Further, the present invention provides a device for applying a powder or granule, comprising a supply unit of the powder or granule, and a conveyor unit for conveying and applying the powder or granule from the supply unit, wherein the conveyor unit has a screw feeder for pulling out the powder or granule from an inside of the supply unit, and a vibrating conveyor unit for conveying the pulled-out powder or granule while dispersing the powder or granule by vibration, characterized in by comprising a first measuring means for continuously measuring the total mass of the supply unit, the screw feeder and the powder or granule therein; and a screw control unit for controlling the screw rotation speed of the screw feeder, wherein the screw control unit calculates the decrement of the powder or granule per time in the screw feeder based on a plurality of measured values by the first measuring means, and controls the screw rotation speed of the screw feeder so as to allow the decrement per time to coincide with a target discharge amount per time.

Further, the present invention provides a method for manufacturing a heat generating element, wherein a step for applying a powder or granule of electrolyte, by applying the method for applying the powder or granule, on the base material to be continuously conveyed, and a step for coating a coating composition containing no electrolyte and containing oxidizable metal particles and water are applied, in this order, in a reverse order or simultaneously.

Other and further objects, features and advantages of the invention will appear more fully from the following description.

BRIEF DESCRIPTION OF DRAWINGS

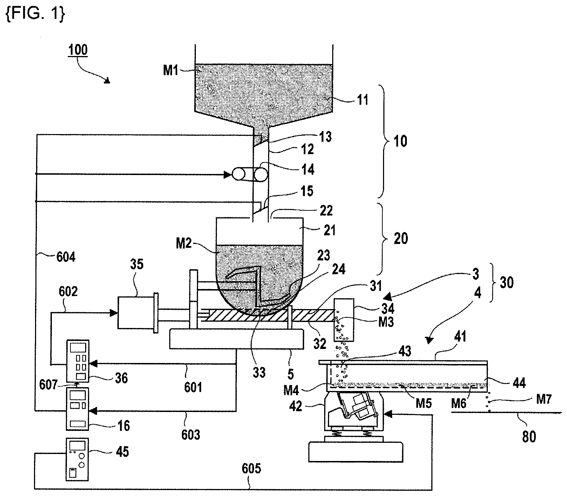

{FIG. 1}
FIG. 1 is a configuration diagram showing one preferred embodiment (first embodiment) of a device for applying a powder or granule of the present invention.
{FIG. 2}
FIG. 2 is a configuration diagram showing a positional relationship between a screw feeder and a vibrating conveyor unit in FIG. 1.
{FIG. 3}
FIG. 3 is a partially enlarged perspective view showing an aspect of delivery of the powder or granule from a screw feeder to a vibrating conveyor unit in the first embodiment.
{FIG. 4}
FIG. 4 is a flowchart showing a method for applying the powder or granule using the device of the first embodiment.
{FIG. 5}
FIG. 5 is an explanatory diagram schematically showing a manufacturing step as one preferred embodiment of a method for manufacturing a heat generating element according to the present invention.
{FIG. 6}
FIG. 6 is an explanatory diagram schematically showing an aspect of applying the powder or granule in the case where heat generating elements are arranged in a line in a cross direction in a method for manufacturing a heat generating element in FIG. 5.
{FIG. 7}
FIG. 7 is a configuration diagram showing another preferred embodiment (second embodiment) of a device for applying the powder or granule of the present invention.
{FIG. 8}
FIG. 8 is a partially enlarged perspective view showing an aspect of delivery of the powder or granule from a screw feeder to a vibrating conveyor unit in the second embodiment.
{FIG. 9}
FIG. 9 is a configuration diagram showing further another preferred embodiment (third embodiment) of a device for applying the powder or granule of the present invention.
{FIG. 10}
FIG. 10 is a flowchart showing a vibration control method in a vibrating conveyor unit according to the third embodiment.

DESCRIPTION OF EMBODIMENTS

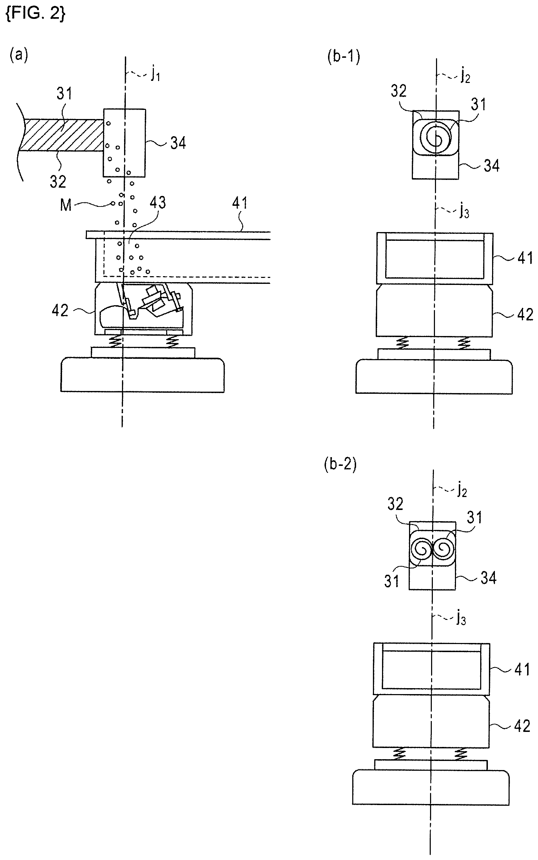

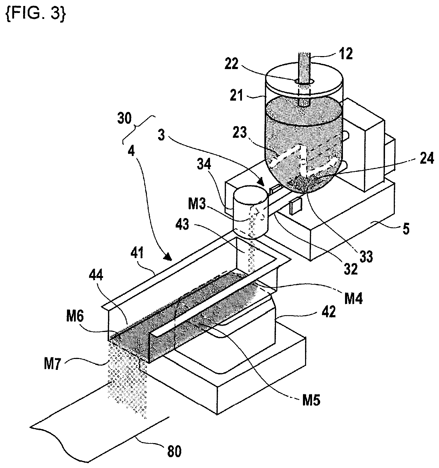

When a powder or granule to be applied is easily solidified under slight pressure, highly deliquescent like salt (sodium chloride) containing magnesium chloride, or highly hygroscopic like a superabsorbent polymer, it is occasionally coagulated by mechanical pressure or absorption of moisture in the air in a conveyance step. In particular, when the conveyance step is carried out using a screw feeder, the powder or granule lumps occasionally bite onto the screw to cause fluctuation of the rotational speed. The clipping amount per time by the screw feeder is determined by the volume of the powder or granule that can be held by the screw (dependent on screw inter-pitch distance or channel depth), and the screw rotational speed, so that fluctuation of the rotational speed disturbs continuous quantitative delivery of the powder or granule. Under such circumstances, unevenness arises in an amount of applying the powder or granule to the base material to be continuously conveyed, which is unfavorable.
Therefore, the present invention relates to a method and a device for applying a powder or granule, which enables the amount of applying the powder or granule on the base material being continuously conveyed to be uniformized and controlled, and a method for manufacturing a heat generating element using the same.
The present invention is described below based on one preferred embodiment.
Firstly, one preferred embodiment (first embodiment) of an application device used for a method for applying a powder or granule according to the present invention is described below, referring to FIG. 1 and FIG. 2. FIG. 1 is a configuration diagram showing a device 100 for applying the powder or granule according to the first embodiment. FIG. 2 is a configuration diagram showing a positional relationship between the screw feeder and the vibrating conveyor unit in the device shown in FIG. 1. FIG. 3 is a partially enlarged perspective view showing an aspect of delivery of the powder or granule from the screw feeder to the vibrating conveyor unit in the first embodiment.
The device 100 for applying the powder or granule according to the embodiment is broadly classified into a stock unit 10, a supply unit 20 and a conveyor unit 30.
The stock unit 10 is arranged upstream (on a upper side) of the supply unit 20, and has a storage tank 11, a distribution pipe 12 of the powder or granule, and a first butterfly valve 13, a rotary valve 14 and a second butterfly valve 15 in the distribution pipe 12. An accumulated powder or granule M1 within the storage tank 11 is distributed to the supply unit 20 on a lower side through the distribution pipe 12. An amount and timing of distribution thereof are adjusted by opening and closing of the first butterfly valve 13, the rotary valve 14 and the second butterfly valve 15.

The supply unit 20 is constituted of a hopper for supplying part of the accumulated powder or granule M1 to the conveyor unit 30 while temporarily storing part of the powder or granule M1 (hereinafter, the supply unit 20 is also referred to as the hopper 20). The hopper 20 is equipped with a storage body unit 21, and a distribution receiving opening 22 of the accumulated powder or granule M1, an agitator 23 for agitating a storage powder or granule M2 in the storage body unit 21, and a supply opening 24 of the storage powder or granule M2, to the storage body unit 21. As shown in FIG. 1, in the supply unit 20, the distribution pipe 12 of the stock unit 10 is inserted into the distribution receiving opening 22 so as for the powder or granule to be distributed from the stock unit 10, but the distribution pipe 12 and the distribution receiving opening 22 are not jointed. The supply opening 24 is an opened part being lack of outer wall in a hemispherical bottom of the storage body unit 21. A screw 31, as described below, enters into this position, and is configured to be brought into direct contact with the powder or granule M2. The hopper 20 receives the accumulated powder or granule M1 to be distributed from the above distribution pipe 12 through the distribution receiving opening 22 to allow storage of the powder or granule as the storage powder or granule M2 in the storage body unit 21 up to a predetermined amount (predetermined height). During operation of the application device 100, the later-mentioned screw feeder 3 rotates to supply the storage powder or granule M2 in the hopper 20 to the screw feeder 3 through the supply opening 24 every in a fixed amount. Moreover, during the operation, the agitator 23 rotates to agitate the storage powder or granule M2 in the hopper 20.

In the hopper 20, as shown in FIG. 1, part of the accumulated powder or granule M1 piles up in a manner of falling from the upward distribution receiving opening 22, and the storage powder or granule M2 is pulled out every in a fixed amount from the downward supply opening 24 by propulsion under deposition pressure of the piled-up storage powder or granule M2. In this case, a shape of the hopper is preferably tapered to be narrowed toward the supply opening so as to avoid excessive output of the storage powder or granule M2 while utilizing pressure. Moreover, the storage powder or granule M2 is preferably supplied sequentially from distributed one in a viewpoint of keeping quality of the powder or granule. In particular, when the powder or granule is highly deliquescent or hygroscopic, the powder or granule preferably avoids continuously staying inside the hopper so as to cause no coagulation. Specific examples of phenomena for disturbing normal supply of the powder or granule include a funnel flow state in discharge of the powder or granule or formation of a bridge or a rat hole within the storage body unit 21. As this resolution, agitation by the agitator 23 is effective. In addition, driving of the agitator 23 may be made in the later-mentioned driving unit 35 of the screw feeder, or an independent driving unit (not shown) separately arranged for the agitator 23.

The conveyor unit 30 has the screw feeder 3 for continuously pulling out the storage powder or granule M2 in the supply unit 20 and a vibrating conveyor unit 4 for gradually dispersing the pulled out powder or granule M3 to convey them by vibration (see FIG. 3).

The screw feeder 3 has a spiral screw 31, an outer wall section 32 for covering the screw 31, an intake 33 of the storage powder or granule M2, a cylindrical outlet 34, a driving unit 35 for rotating the screw 31, and a screw control unit 36 for controlling the driving unit 35. The screw 31 has length in a substantial horizontal direction from the intake 33. The screw 31 is connected with the driving unit 35 and rotates with the power. The storage powder or granule M2 in the supply unit 20 is subdivided and pulled out by rotation of this spiral screw 31, and conveyed in a longitudinal direction of the screw within the outer wall section 32. Further, the powder or granule M3 is continuously discharged from the outlet 34 in the screw end. The outlet 34 is projected downward of a lower end of the outer wall section 32 to facilitate falling of the powder or granule M3 in a vertical direction.

The subdivision and the pulling-out of the powder or granule by the spiral screw 31 enable the storage powder or granule M2 to be prevented from being instantaneously pushed out by their own deposition pressure, and enable control of a discharge amount of the pulled-out powder or granule M3 to the vibrating conveyor unit 4. Thus, the subdivision and the pulling-out facilitate dispersion in the vibrating conveyor unit 4 to allow effective dispersion. An amount ($\Delta K$) of the powder or granule M3 discharged from the outlet 34 per time ($\Delta T$) is mainly influenced by a screw rotational speed (P), or the like. The rotational speed (set value) ($P_0$) of the screw 31 is set up according to a target discharge amount ($\Delta K_0$) of the powder or granule M3 per time ($\Delta T$). An actual rotational speed (P) is stabilized along with the rotational speed ($P_0$), and thus uniformization of the pulled out amount may be held. However, when powder or granule to be pulled out is deliquescent or hydroscopic, for example, coagulation or deliquescence takes place even in the rotating screw feeder to easily form a lump. The screw 31 bites on the powder or granule lump to cause delay of the rotational speed. In this case, the screw 31 tends to crush a bitten lump to decrease the rotational speed. Conversely, after crushing, the screw 31 becomes light by the reaction force, and the rotational speed temporarily rises. As a result, the rotational speed of the screw 31 fluctuates to cause unevenness over time in the discharge amount ($\Delta K$) of the powder or granule M3 per time ($\Delta T$), and thus uniformization is disturbed. Fluctuation of the discharge amount ($\Delta K$) of the powder or granule M3 per time ($\Delta T$) by an increase and a decrease in the rotational speed of the screw depends on a time division manner, but when the time is divided in a unit of 1 second or 0.2 second, for example, application with higher accuracy is required for the base material to be continuously conveyed in a production line in a plant.

Consequently, in the device 100 of the embodiment, the conveyor unit 30 is formed in combination with the screw feeder 3 and the vibrating conveyor unit 4 to enable resolution of discharge unevenness of the pulled out powder or granule M3 to uniformize the discharge, and to suitably apply the powder or granule M3 on the base material 80 with causing no recoagulation.

The vibrating conveyor unit 4 has a trough 41 as a conveyor element for dispersing and conveying powder or granule M4 in a fallen state of the powder or granule M3, a vibrating element 42 for vibrating the trough 41, and a vibration control unit 45. The vibrating conveyor unit 4 is separated from the outlet 34 of the screw feeder 3 and positioned on a lower side. The trough 41 has an oblong shape along a conveyance direction of the powder or granule, and has a recess opened above. The vibrating element 42 is arranged below one end side of the trough 41, and both are fixed in the position. The outlet 34 of the screw feeder 3 is arranged above the trough 41 in which the vibrating element 42 is arranged. One end side of this trough 41 serves as a receiving position 43 of the powder or granule M3 falling from the outlet 34. A powder or granule M4, which is received in the receiving position 43, is vibrated and dispersed together with vibration of the trough 41 by the vibrating element 42, and advances in a horizontal direction to the other end side of trough 41. The other end of this trough 41 serves as a sprinkling outlet 44 for uniformly applying a powder or granule M7 uniformly dispersed by vibration of the vibrating element 42 on the base material 80 to be continuously conveyed. The width of the trough 41 and the width of the sprinkling outlet 44 are broadly formed in comparison with the width of the outlet 34 of the screw feeder. The trough 41 is in a cantilevered state with the trough 41 fixed to the vibrating element 42 at the receiving position 43, and not fixed at the sprinkling outlet 44. Thus, the vibration of the vibrating element 42 is gradually amplified and propagated from the receiving position 43 of the trough 41 to the sprinkling outlet 44, and the powder or granule M4 on the trough 41 moves on to the sprinkling outlet 44 while being suitably dispersed. Then, the powder or granule is applied onto the base material 80 as the uniformly dispersed and smoothly leveled the powder or granule M7. Moreover, the width of the trough 41 and the width of the sprinkling outlet 44 of the vibrating conveyor unit 4 are broadly formed in comparison with the width of the outlet 34 of the screw feeder, and thus the powder or granule fed out in a fixed amount by the screw 31 diffuses in a width direction to be uniformly applied. The base material 80 is in a sheet form and the width is broadly formed in comparison with the width of the sprinkling outlet 44. Hereinafter, the base material 80 is also referred to as a base material sheet 80.

In the present embodiment, the receiving position 43 of the trough 41 and the vibrating element 42 are arranged with the outlet 34 in line with a vertical line ($j_1$) (see FIG. 2(a)). This arrangement allows combination of falling impact force by self-weight of the powder or granule M3 fed out by the screw 31 and force of vibration by the vibrating element 42 to produce synergistic dispersion force. This arrangement is effective for dispersing the powder or granule M4 on the trough 41. In addition, presence of the receiving position 43 of the trough 41 and the vibrating element 42, and the outlet 34 in line with the vertical line also includes substantial presence of the receiving position 43 of the trough 41 and the vibrating element 42, and the outlet 34 in line with the vertical line. The substantial presence in line with the vertical line means an arrangement in which the receiving position 43, the vibrating element 42 and the outlet 34 are overlapped so as to enable the synergistic dispersion force by falling and vibration to be effectively produced. In this case, an upper surface center point of the vibrating element 42 does not need to be in line with the vertical line with the outlet 34 and the receiving position 43, and a partially overlapped arrangement of a degree at which the vibration is effectively transmitted is sufficient.

Further, in a front view of the trough 41 along a direction perpendicular to the direction of conveying the powder or granule from the receiving position 43 of the vibrating conveyor unit 4 to the sprinkling outlet 44, the arrangement is configured so that a width central position of the receiving position 43 coincides with a width central position of the outlet 34 of the screw feeder. In addition, the direction perpendicular to the conveying direction means a direction perpendicular to the conveying direction in an identical horizontal plane, and means a CD (Cross Direction) when a flow direction of the powder or granule is taken as an MD (Machine Direction). More specifically, as shown in FIG. 2(b-1), a center line $j_3$ of width of the receiving position 43 of the trough 41 of the vibrating conveyor unit 4 coincides with the center line $j_2$ of width in the vicinity of the outlet 34. Thus, the powder or granule M4 that starts dispersion by the above-described impact easily diffuses uniformly in the cross direction (CD) on the trough 41, which is preferred. The screw 31 is not limited to a single screw, and may include a pair of screws or more screws. Even in such a case, as shown in FIG. 2(b-2), a center line $j_4$ of the width of the outlet 34 in combination with a plurality of screws coincides with a center line $j_3$ of the width of the receiving position 43. In addition, coincidence of the width central position of the receiving position 43 with the width central position of the outlet 34 of the screw feeder includes a case where the width central position of the receiving position 43 substantially coincides with the width central position of the outlet 34 of the screw feeder. Substantial coincidence means that the two positions substantially coincide with each other in a range in which the powder or granule M4 is received in the vicinity of the center in the cross direction of the receiving position 43.

The trough 41 in the embodiment has a rectangular parallelepiped shape. However, as the conveyor element according to the present invention, the shape is not limited thereto, and a shape suitably dispersing the powder or granule from the receiving position 43 to allow uniform application in the sprinkling outlet 44 can be arbitrarily applied. For example, as a shape in the planar view of the conveyor element, the conveyor element may have a trapezoidal shape, a shape in which width is enlarged as approaching to the sprinkling outlet 44 from the receiving position 43, or a shape in which the width is conversely narrowed. Moreover, the conveyor element as a whole may be formed into a circular arc shape. Further, all upper parts of the trough 41 in the embodiment are opened, but the configuration is not limited thereto, and an upper part other than the receiving position 43 may be closed.

The vibration of the vibrating element 42 is adjusted by amplitude (r) and frequency (h) control using the vibration control unit 45 (see symbol 605 in FIG. 1). If the amplitude and the frequency of vibration (changed by the frequency) are excessively large, the powder or granule arrives at the sprinkling outlet 44 without being sufficiently dispersed. Conversely, if the amplitude and the frequency of vibration are excessively small, the powder or granule is quite difficult to move forward on the trough 41, and stagnate. A suitable setup of the amplitude (r) and the frequency (h) of the vibrating element 42 can be arbitrarily made based on a relation with length ($n_1$) and width ($n_2$) of the trough 41. The relation of the length ($n_1$) and the width ($n_2$) of the trough 41, and the amplitude (r) and the frequency (h) of the vibrating element 42 differs also depending on a required level of the application amount of the powder or granule, and therefore is not uniquely determined. However, the frequency (h) of the vibrating element 42 is preferably set up so as to coincide with the natural frequency of the trough 41 so as to most efficiently vibrate the trough 41, and then the amplitude (r) of the vibrating element 42 is preferably set up so as to complete uniform dispersion of the powder or granule in the vicinity of the sprinkling outlet 44 of the trough 41. Such a configuration avoids stagnation of the powder or granule on the trough 41 and enables application of the uniformly dispersed powder or granule onto the base material 80.

In addition, the natural frequency of the trough 41 serving as a standard of the above-mentioned frequency (h) setup can be measured by applying a method used for this kind of device, for example, can be measured by combining an accelerometer and an FFT (Fast Fourier transform) analyzer. More specifically, the accelerometer is attached to a leading end of the trough 41 to collect vibration waves, and FFT frequency analysis is conducted by the FFT analyzer. Thus, a wavelength peak appears in the vicinity of the natural frequency. Moreover, as a method for setting up the amplitude (r) so as to complete the uniform dispersion of the powder or granule in the vicinity of the sprinkling outlet 44, for example, the adjustment may be conducted on the basis of direct visual observation, or by detecting the powder or granule falling from the sprinkling outlet 44 with a CCD camera, the amplitude may be adjusted so as to uniformize a pixel distribution of the powder or granule in the cross direction. Further, in order to achieve still more effective vibration of the trough 41 by the vibrating element 42, the powder or granule on the trough 41 is preferably as little (light) as possible so as to reach the sprinkling outlet 44 as soon as possible without stagnation. Therefore, a state in which the powder or granule is completely dispersed widely in the vicinity of the sprinkling outlet 44 amounts to a state in which the powder or granule is uniformly dispersed while most quickly reaching the state, which is preferred. Specific examples of the setup method therefor include a method of starting with high amplitude at which the powder or granule cannot be completely dispersed and gradually reducing the amplitude (r), thereby establishing the amplitude (r) that completely disperses the powder or granule in the vicinity of the sprinkling outlet 44. When an application amount is linearly corrected after setup of the frequency (h) and the amplitude (r), linear adjustment is further effected by adjusting the amplitude (r) of the vibrating element 42 rather than adjusting the frequency (h) of the vibrating element 42, in which a degree of vibration is significantly influenced by the natural frequency of the trough 41, and therefore such adjustment is preferred.

As described above, the combination of the screw feeder 3 and the vibrating conveyor unit 4 is suitable for uniform application of the powder or granule in the application device 100 for the powder or granule in the embodiment. More specifically, the screw feeder 3 subdivides and draws out the powder or granule in the hopper 20 to moderately relax pushing pressure of the powder or granule, thereby facilitating subsequent dispersion. Further, even if fluctuation of a discharge amount ($\Delta K$) of the powder or granule M3 by a fluctuation or the like of the rotational speed ($P_0$) of the screw 31 arises, the vibrating conveyor unit 4 subsequently receives the fluctuation to effectively disperse the powder or granule by the vibration so as to adjust the fluctuation, thereby allowing uniform application. Moreover, arrangement of the outlet 34 of the screw feeder 3 and the receiving position 43 of the vibrating conveyor unit 4 causes easy and direct transmission of the vibration of a vibration unit 42 in the vibrating conveyor unit 4 to the fallen powder or granule M4, and thus the powder or granule may be uniformly dispersed in the width direction of the trough 41 without unevenness (a dispersed powder or granule M5 to M6). In addition thereto, the cantilever structure of the trough 41 amplifies the vibration in the conveyance direction, and thus dispersion and conveyance of the powder or granule are realized with adequate balance. Thus, stagnation or coagulation of the powder or granule M4 is prevented, and quick and efficient uniform application of the powder or granule M7 is realized. Further, the setup in combination of the rotational speed ($P_0$) with the amplitude ($r_0$) and the frequency ($h_0$) without setting up only the rotational speed ($P_0$) makes it possible to achieve uniform application of the powder or granule with higher accuracy in response to various required levels of application amount.

Further, in the application device 100 for the powder or granule according to the embodiment, the device has a control mechanism as described below, whereby further accurate sprinkle of the powder or granule M7 can be achieved, which is preferred. This point is described below.

The application device 100 has a first measuring means 5. The first measuring means 5 is electrically connected with a screw control unit 36 and a powder or granule supply amount control unit 16. As the first measuring means 5, an electric meter can be used, for example. Specifically, a load-cell meter, an electromagnetic meter, a tuning fork meter or the like can be used.

The first measuring means 5 continuously measures the supply unit 20, the screw feeder 3, and a total mass ($K_t$) of the powder or granule therein. The screw control unit 36 receives a plurality of continuously measured data from the first measuring means 5 (see symbol 601 in FIG. 1), and on a basis thereof, calculates a change rate of total mass ($K_t$) of a measured value relative to time, more specifically, a decrement (discharge amount) ($\Delta K_t$) of the powder or granule M3 per time ($\Delta T$). Then, the screw control unit 36 compares a calculated decrement (discharge amount) ($\Delta K_t$) with a predetermined target discharge amount ($\Delta K_0$) of the powder or granule M3 per time ($\Delta T$) to give an instruction to the driving unit 35 to control the rotational speed of the screw 31 so as for both to coincide with each other (see symbol 602 in FIG. 1). For example, in case of $\Delta K_t > \Delta k_0$, the rotational speed ($P_t$) of the screw 31 is decreased to suppress the discharge amount, and in case of $\Delta K_t < \Delta K_0$, the rotational speed ($P_t$) of the screw 31 is increased to raise the discharge amount.

The decrement ($\Delta K_t$) of the powder or granule M3 per time ($\Delta T$) is preferably calculated, for example, based on the following Formula (1). Further, allowable range of a deviation $\varepsilon$ between $\Delta K_t$ and $\Delta K_0$ (= $\Delta K_t$ - $\Delta K_0$) is preferably lower than ±1% of the target discharge amount $\Delta K_0$ in terms of a mass ratio, and an increase and a decrease in the rotational speed when a deviation $\varepsilon$ exceeding this range arises is preferably performed based on the following Formula (2). The following Formula (2) corresponds to a PI (Proportional Integral) control expression, and the rotational speed ($P_t$) of the screw 31 is controlled so as for the deviation $\varepsilon$ to be 0 (zero). Specifically, when the deviation $\varepsilon$ arises, the rotational speed is instantaneously corrected by $100/K_P$ times of the deviation $\varepsilon$ (hereinafter, referred to as proportional action), and correction in a value identical with the correction of the rotational speed by the proportional action is executed by multiplying the time of an integration constant $K_I$. In the PI control, a correction amount at a fixed ratio is first added to the deviation in the P-action (hereinafter, referred to as an offset). A residual deviation from the target value remains only by the offset in the P-action, and therefore adjustment is made so as to eliminate the remaining deviation from the target value in I-action. This adjustment is effective in suppressing the fluctuation of pulling out amount in the screw feeder.

$$\Delta K_t = (K_{tn} - K_{t(n+\Delta T)}) \qquad \text{Formula (1)}$$

$K_{tn}$ = total mass of a measured value at a certain time point
$K_{t(n+\Delta T)}$ = total mass of a measured value at a time point after elapse of $\Delta T$ from $K_{tn}$

$$\text{Change amount of rotational speed (\%)}$$
$$= (100/K_P)*\{\varepsilon+(1/K_I)\textstyle\int\varepsilon dt\} \qquad \text{Formula (2)}$$

$K_p$ = proportional constant
$K_I$ = integration constant
$\varepsilon = \Delta K_t - \Delta K_0$

Moreover, the powder or granule supply amount control unit 16 receives a plurality of data continuously measured by the first measuring means 5 (see symbol 603 in FIG. 1) to keep track of a decrease transition of the total mass (Kt) of the supply unit 20, the screw feeder 3 and the powder or granule therein. On the basis thereof, the unit 16 judges necessity of distribution of the powder or granule to the hopper 20. Specifically, when a measured value is lower than a target lower limit amount ($K_{min}$), the unit 16 distributes the powder or granule from the stock unit 10 and stops the distribution at a target upper limit amount ($K_{max}$). This distribution of the powder or granule is made by control of the rotary valve 14, the first butterfly valve 13 and the second butterfly valve 15 of the stock unit 10 by the powder or granule supply amount control unit 16 (see symbol 604 in FIG. 1). In addition, in order to avoid inclusion of a mass of the stock unit 10 in each measured value ($K_t$), the hopper 20 and the distribution pipe 12 of the stock unit 10 are configured to be in a non-contact state.

Keeping track of a decrease of this total mass ($K_t$) leads to indirectly keeping track of a remaining amount of the stock powder or granule M2 in the hopper 20 and a deposition level of the stock powder or granule M2 in the hopper 20. This deposition level indicates a deposition pressure level of the powder or granule M2 to indicate a level of ease of pulling out the powder or granule by the screw feeder. Accordingly, the target lower limit amount ($K_{min}$) indicates a lower limit amount of the total mass ($K_t$) corresponding to an allowable deposition level in pulling out by the screw feeder. Moreover, the target upper limit amount ($K_{max}$) indicates an upper limit amount of the total mass ($K_t$) corresponding to the allowable deposition level. From this viewpoint, the target lower limit amount ($K_{min}$) is adjusted to a value obtained by adding, to a total ($K_P$) of a mass of devices per se of the supply unit 20 and the screw feeder 3 and a fixed amount of the powder or granule taken in the screw feeder, preferably, a mass of the powder or granule corresponding to 2 % or more and 40% or less of the volume of the hopper 20, and further preferably, a mass of the powder or granule corresponding to 30 % or more and 40% or less. Moreover, the target upper limit amount ($K_{max}$) is adjusted to a value obtained by adding, to ($K_P$), preferably, a mass of the powder or granule corresponding to 50% or more and 100% or less, and further preferably, a mass of the powder or granule corresponding to 50 % or more and 60% or less. In addition, the above-mentioned "fixed amount of the powder or granule taken in the screw feeder" means a mass of the powder or granule all the time existing in the screw feeder even during operation of the application device for the powder or granule. Moreover, the volume of the hopper 20 means a mass of the powder or granule that can be put into the hopper 20 without including the weight of the hopper per se.

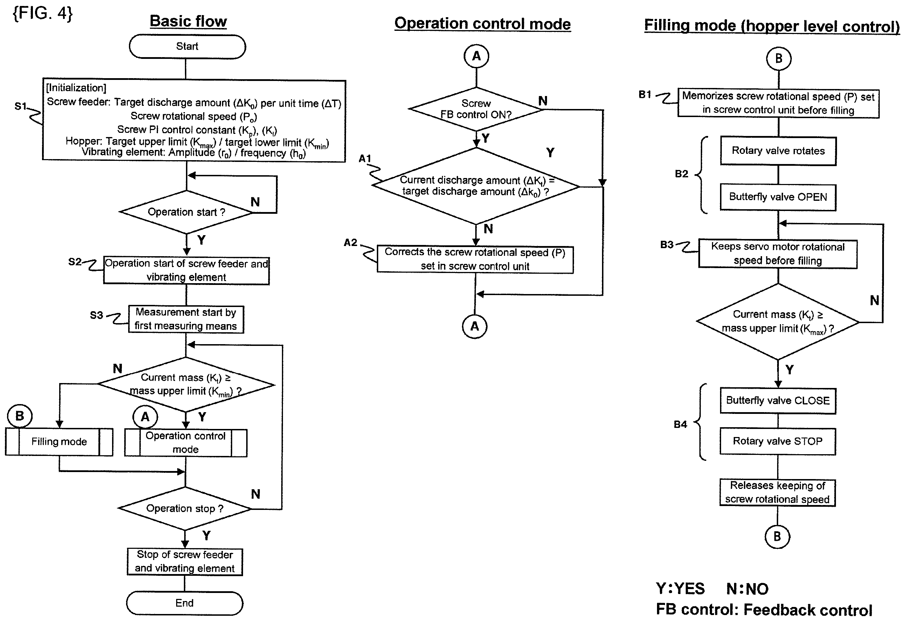

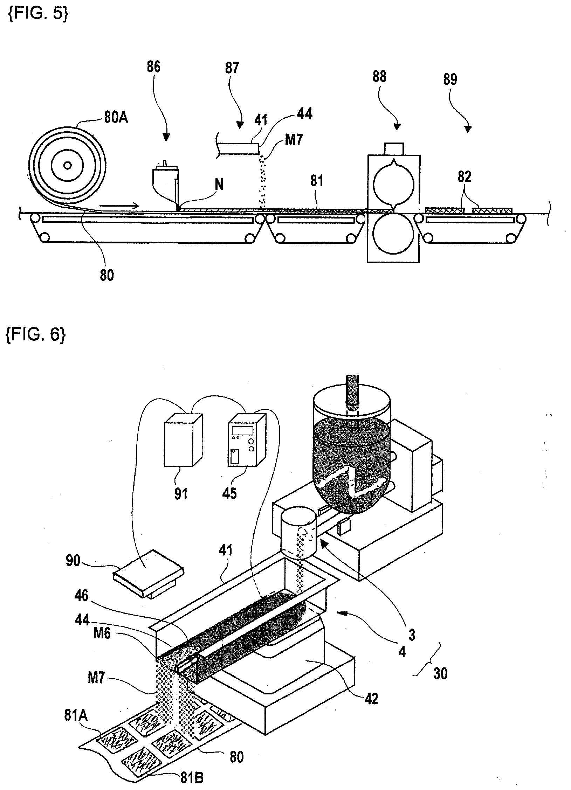

Next, a preferred embodiment of a method for applying a powder or granule and a method for manufacturing the heating element applying the method for applying the powder or granule according to the present invention are described in detail referring to FIGs. 1, 4 and 5. FIG. 4 is a flowchart showing a method for applying the powder or granule using an application device 100 for the powder or granule according to a first embodiment. FIG. 5 is an explanatory diagram schematically showing a manufacturing step as one preferred embodiment of a method for manufacturing a heat generating element according to the present invention.

Specific examples of the powder or granule handled herein include an electrolyte having an average particle diameter of 50 $\mu$m or more and 1,000 $\mu$m or less in a solid state to be used for the heat generating element. More specific examples include the powder or granule which can dissolve oxide formed on a surface of a particle of an oxidizable metal contained in the heat generating element. As an example, alkali metal chlorides, alkaline earth metal chlorides or transition metal chlorides is preferably used, and sodium chloride, potassium chloride, calcium chloride, magnesium chloride, ferrous chloride or ferric chloride is particularly preferably used. This electrolyte powder or granule is uniformly applied at a predetermined basis weight on an elongated base material sheet 80 to be continuously conveyed. A heat generating device formed of the heat generating element to be manufactured applying such an electrolyte is suitably used for warming mainly a specific site (shoulder, neck, face, eyes or the like) of a body, and an area is relatively small. Incorporation of the electrolyte into this small heat generating element at a predetermined basis weight becomes essential to product quality such as heating characteristics (stable continuation of predetermined temperature, shortage of rise time to a predetermined temperature, or the like) of the heat generating element. The basis weight of the electrolyte in the case of the heat generating element is, for example, 4 $g/m^2$ or more and 80 $g/m^2$ or less, and particularly, 5 $g/m^2$ or more 30 $g/m^2$ or less. Furthermore, the heat generating element is obtained by cutting one manufactured on the base material sheet 80 to be continuously conveyed into small pieces, and therefore the electrolyte should be applied by accurately uniformizing the application basis weight per time.

The method for applying the powder or granule according to the embodiment is suitable for uniformization of the application amount for highly deliquescent or hygroscopic powder or granule that is easily coagulated in the convey step. Moreover, according to the method for manufacturing the heat generating element applying this application method therefor, the heat generating element allowing uniformization of the heating characteristics can be suitably obtained.

The method for applying the powder or granule according to the present invention is first described based on the method for applying the powder or granule using the application device 100 for the powder or granule.

In the embodiment, as shown in a basic flow in FIG. 4, numerical value setup for uniform application of the powder or granule M3 is input into each control unit (step S1). Specifically, the target discharge amount ($\Delta K_0$) of the powder or granule M3 per time ($\Delta T$), the rotational speed ($P_0$) of the screw 31, and the proportional constant ($K_P$) and the integration constant ($K_I$) to be used for PI control are input into the screw control unit 36. Moreover, the target lower limit amount ($K_{min}$) and the target upper limit amount ($K_{max}$) are input into the powder or granule supply amount control unit 16. Further, the amplitude ($r_0$) and the frequency ($h_0$) of the vibrating element 42 are input into the vibration control unit 45. In this case, the rotational speed ($P_0$) of the screw 31 is set up to be the target discharge amount ($\Delta K_0$) of the powder or granule M3 per time ($\Delta T$), and then the amplitude ($r_0$) and the frequency ($h_0$) of the vibrating element 42 to suitably disperse the powder or granule to allow sprinkle thereof are set up for the target discharge amount ($\Delta K_0$).

Next, based on the input setup value described above, the screw control unit 36 and the vibration control unit 45 are operated. The screw control unit 36 allows the screw to start rotation through the driving unit 35 (symbol 602 in FIG. 1), and the vibration control unit 45 allows the vibrating element 42 to start vibration (symbol 605 in FIG. 1) (step S2). On the occasion, the driving unit 35 allows the agitator 23 of the supply unit 20 to rotate in interlocking with the rotation of the screw 31. When the device 100 has an independent driving unit for driving the agitator 23, the driving unit allows the agitator 23 to rotate so as to interlock with the rotation of the screw 31.

Further, measure of the supply unit 20 and the screw feeder 3 by the first measuring means 5 is started (step S3). In addition, in the embodiment, this start of the measure by the first measuring means 5 is shown to be made after the above step 2, but the start is not limited thereto, and may be simultaneously made, or the measure may be carried out before the step S2. In any of these cases, the start may be made at timing allowing suitable measurement of the total mass of the supply unit 20 and the screw feeder 3, including the powder or granule during an initial stage of operation start.

Unless the measured value ($K_t$) by the first measuring means 5 is lower than the target lower limit amount ($K_{min}$) input in the step S1, the screw control unit 36 allows the screw 31 to keep the rotational speed ($P_0$) thereof. On the other hand, when control is made so as to keep the screw rotational speed ($P_0$), as mentioned above, the fluctuation of the screw rotational speed by the powder or granule lump may arise. In the present invention, even in this case, the conveyor unit 30 is in a step of combination of the screw feeder 3 with vibrating conveyor unit 4, and combination of the rotational speed ($P_0$) of the screw 31 with the amplitude ($r_0$) and the frequency ($h_0$) of the vibrating element 42 allows uniformization of the application amount of the powder or granule. In particular, in the present invention, the powder or granule M3 pulled out from the outlet 34 of the screw feeder 3 is allowed to directly fall to directly vibrate by the vibrating element 42 right below the receiving position 43 of the trough 41 (see FIG. 2). Thus, the impact force caused by falling and the vibration force are effectively combined to further facilitate dispersion of the powder or granule at process from the powder or granule M3 to the powder or granule M4. Further, the width central position of the screw feeder 3 is arranged to substantially coincide with the width central position of the trough 41 of the vibrating conveyor unit 4 (see FIG. 2), and therefore uniform dispersion is facilitated without causing unevenness of the powder or granule M4 in any side of the cross direction. Further, the vibrating conveyor unit 4 allows the powder or granule M4 pulled out and fallen by the screw feeder 3 to vibrate and disperse at the process from the powder or granule M5 to the powder or granule M6 immediately before sprinkling. Thus, the combination of the screw feeder 3 and the vibrating conveyor unit 4 allows uniform application of even the highly deliquescent or hydroscopic powder or granule, without occurrence of coagulation of the powder or granule M7, on the base material 80 to be continuously conveyed.

In addition thereto, as shown in an operation control mode (A) in FIG. 4, if the screw control unit 36 performs feedback control for adjusting the rotational speed ($P_0$) of the screw 31 to the above fluctuation based on the measured value ($K_t$), the fluctuation of the discharge amount of the powder or granule by the screw feeder 3 is also suppressed, which is preferred. Specifically, as mentioned above, the screw control unit 36 compares the discharge amount ($\Delta K_t$) of the powder or granule M3 per time ($\Delta T$), which is calculated based on the measured value ($K_t$) (symbol 601 in FIG. 1) received from the first measuring means 5, with the target discharge amount ($\Delta K_0$) of the powder or granule M3 per time (AT) (step A1), and in case of $\Delta K_t < \Delta K_0$, the screw control unit 36 gives a correction instruction to the driving unit 35 so as to increase the screw rotational speed, and in case of $\Delta K_t > \Delta K_0$, to decrease the screw rotational speed (sign 602 in FIG. 1) (step A2). Thus, the actual screw rotational speed is brought close to the target rotational speed ($P_0$) of the screw 31, and is stabilized to allow discharge of the powder or granule in the target discharge amount ($\Delta K_0$) of the powder or granule M3 per time (AT). Then, dispersion in the vibrating conveyor unit 4 also becomes further effective. The feedback control by this screw control unit 36 is repeatedly performed during operation of the application device 100.

In addition thereto, in the application method according to the embodiment, as shown in a filling mode (hopper level control) (B) in FIG. 4, the deposition level of powder or granule in the hopper 20 is controlled by the powder or granule supply amount control unit 16 so as not to be lower than the target lower limit amount ($K_{min}$) affecting subdivision and pulling-out thereof. Specifically, first, if the measured value ($K_t$) (= total mass of the supply unit 20, the screw feeder 3 and the powder or granule therein) (symbol 603 in FIG. 1) received from the first measuring means 5 is lower than the target lower limit amount ($K_{min}$), the screw rotational speed is memorized in the screw control unit 36 by an instruction from the powder or granule supply amount control unit 16 (symbol 607 in FIG. 1) (step B1).

Further, the powder or granule supply amount control unit 16 causes rotation of the rotary valve 14 of the stock unit 10 to open the butterfly valves 13 and 15 to supply the powder or granule to the hopper 20 (symbol 604 in FIG. 1) (step B-2). On the occasion, the screw control unit 36 causes to keep the above memorized screw rotational speed (step B3). Accordingly, the powder or granule supply amount control unit 16 controls the supply amount from the stock unit 10 so as to be higher than the pulling-out amount of the powder or granule M3 by the screw 31. Moreover, in the meantime, the powder or granule supply amount control unit 16 receives the measured value ($K_t$) by the first measuring means 5 (symbol 603 in FIG. 1), and at a time point when the measured value ($K_t$) reaches or becomes higher than the target upper limit amount ($K_{max}$), the control unit 16 causes closure of the butterfly valves 13 and 15 to stop rotation of the rotary valve 14 (symbol 604 in FIG. 1) (step B4). At the time point, the screw control unit 36 releases keeping of the screw rotational speed in the step B3 based on the instruction from the powder or granule supply amount control unit 16 (symbol 607 in FIG. 1).

This hopper level control by the powder or granule supply amount control unit 16 is repeatedly performed during the operation of the application device 100, regardless of presence or absence of the above-mentioned feedback control.

Thus, the method for applying the powder or granule according to the embodiment enables uniform application of the powder or granule, particularly, the powder or granule which has highly deliquescent or hydroscopic properties or the powder or granule easily forming the lump by pressure, on the base material 80 to be continuously conveyed without coagulation.

Next, a preferred embodiment of the method for manufacturing a heat generating element utilizing the above method for applying the powder or granule is described referring to FIG. 5. In the method for manufacturing the heat generating element, the above application device 10 for the powder or granule is used.

The heat generating element, which is handled in the method for manufacturing the heat generating element according to the embodiment, is formed by arranging a layer of a heat generating composition (hereinafter, also referred to as a heat generating layer) at least on one surface of the base material sheet 80. The heat generating layer is formed including at least oxidizable metal particles, an electrolyte and water. The heat generating layer may further contain a reaction accelerator. The base material sheet 80 may include one sheet or two sheets, and in the case of two sheets, when the heat generating layer is interposed between both sheets, sticking of the heat generating layer to packaging material can be avoided, which is preferred. As the electrolyte, one exemplified above is used. As the base material sheet 80, one used for this kind of articles can be arbitrarily adopted, and particularly from a viewpoint of control of moisture content, a fibrous sheet including hydrophilic fibers or a fibrous sheet including superabsorbent polymer particles is preferred. Examples of the oxidizable metal contained in the heat generating layer include iron, aluminum, zinc, manganese, magnesium, and calcium. Examples of the reaction accelerator used if necessary include activated carbon (e.g., coconut shell activated carbon, wood activated carbon, bituminous coal, peat, and lignite), carbon black, acetylene black, graphite, zeolite, pearlite, vermiculite, and silica.

In the method for manufacturing the heat generating element according to the embodiment, as shown in FIG. 5, the method comprises the following steps on the continuously elongated base material sheet 80 delivered from an original sheet roll 80A:

(1) a step (coating step 86) for coating a coating composition N containing the oxidizable metal particles and water; and
(2) a step (electrolyte application step 87) for applying electrolyte powder or granule M7.

The heat generating layer 81 is formed on the base material sheet 80 in the above steps (1) and (2). Further, in a cutting-out step 88, cutting-out is made over a width direction in a part without the heat generating layer in case that the coating composition N is intermittently coated, or in a part with the heat generating layer in case that the coating composition N is continuously coated, and in a re-pitch step 89, pitches of ones separated in a front-back direction are aligned to have a fixed interval, and thus a heating element 82 having a predetermined size is obtained. In the above steps (1) and (2), the method may comprise, as a preliminary step of the cutting-out step 88, a step for slitting, into a plurality of rows including two or more rows in the cross direction, the base material sheet 80 on which the heat generating layer 81 is formed. Slitting into the plurality of rows is preferably performed in the case of providing the base material sheet 80 with the coating composition N or the coating composition N and the powder or granule M7 in width substantially identical with the width of the base material sheet 80. The heat generating layer 81 (base material sheet 80) after being slit into the plurality of rows, as necessary, is widened between the rows, and conveyed to the cutting-out step 88.

Thus, the coating composition N containing the oxidizable metal, and the electrolyte powder or granule M7 are incorporated into the base material sheet 80 in different steps, and therefore oxidization of the oxidizable metal in the coating composition is suppressed, and precipitation of agglomerates is prevented, and corrosion of facilities is prevented. Further, the electrolyte includes the powder or granule in a solid state, and therefore excessive addition of moisture to the heat generating element is prevented and favorable control of heat generating characteristics is achieved by applying this electrolyte in the solid state. The coating step (1) and the application step (2) described above may be applied in this order, or may be simultaneously applied, or the step (1) may be applied after the step (2). The applied electrolyte in the solid state is dissolved into water in the coating composition before the time using the heat generating element, and uniformly exists in the heat generating layer of the heat generating element.

Even if the above steps (1) and (2) are applied in any sequential order, the electrolyte powder or granule is required to be in a uniform amount to the above coating composition from a viewpoint of the heat generating characteristics. The powder or granule should be in the uniform amount for a coating region (or entire area of a coating layer) (= heat generating layer 81) or a coating-planned region of the coating composition on the base material sheet 80. From this viewpoint, the above method for applying the powder or granule enables formation of the heat generating element having good heating characteristics in the method for manufacturing the heat generating element.

As a post-step thereof, although not shown, the heat generating element is wrapped with air-permeable wrapping material and sealed. In addition thereto, the sealed product is further cut out to give the heat generating device wrapped in the wrapping material.

In the method for manufacturing the heat generating element according to the embodiment, when the resulting heat generating element is not divided in a width direction (CD (Cross Direction)), application uniformity of the electrolyte powder or granule may be achieved with each other in former and latter coating regions (coating regions with the coating composition in the above step (1)) in a flow direction (MD (Machine Direction)). Even if the electrolyte powder or granule is unevenly applied in one coating region, the powder or granule only needs to be dissolved into the coating layer before use.

On the other hand, when the resulting heat generating element is divided into plurality in the CD direction of the base material sheet 80, the application uniformity is required with each other in the coating regions aligned in the CD direction. For example, as shown in FIG. 6, when two heat generating elements (a heating element A and a heating element B) are aligned and formed in the CD direction, the electrolyte application uniformity is required between a heating layer 81A and a heating layer 81B formed in the positions. In this case, in the above method for applying the powder or granule, a branch plate 46 or the like is disposed in the vicinity of the sprinkling outlet 44 of the trough 41 to disperse the powder or granule in the width direction, thereby enabling uniform dispersion in each amount. Thus, the powder or granule diffused on the trough 41 is branched into a plurality of rows including two or more rows in the sprinkling outlet 44, and continuously applied on the base material sheet 80. In FIG. 6, the coating composition N is intermittently arranged in the flow direction, but may be continuously arranged.

Upon uniformly dispersing the powder or granule in the CD as mentioned above, preferably, a detecting means 90 such as a CCD camera is installed above or below the sprinkling outlet 44 or above the heating layer 81 to detect a dispersion state of the powder or granule, and an information processing unit 91 analyzes the detected data to give an instruction for adjusting the amplitude (r) and the frequency (h) of the vibration to the vibration control unit 45. At this time, for example, the detecting means 90 preferably divides a detection area into plural area in the CD direction and detects dispersion state in each area, and as the detecting means 90, one or a plurality of instruments may be used. Further, the information processing unit 91, for example, determines a ratio at which the powder or granule occupies in each of the above detection area (area ratio (M) = total area of the powder or granule in a detection area/an area of detection area) and compares the thus determined ratios of the powder or granule with each other in the detection areas, and thereby converts the dispersion state of the powder or granule M7 in the CD direction into numerical values. As the numeric conversion method, for example, an average ($M_{aveg}$) is calculated from the area ratio of the powder or granule for every detection area to calculate a ratio ($M/M_{aveg}$) of the area ratio (M) of each detection area based on this average ($M_{aveg}$), and the ratio is taken as a numerical value of the dispersion state.

Based on the numerical value, the information processing unit 91 gets the amplitude (r) and the frequency (h) required for dispersion from preset database to send the information to the vibration control unit 45. The vibration control unit 45 adjusts the amplitude (r) and the frequency (h) based on the information. For example, if the detecting means 90 detects a state in which the powder or granule M6 is excessively accumulated in the vicinity of the branch plate 46, and is not uniformly dispersed in the width direction in the sprinkling outlet 44, the information processing unit 91 gives an instruction for decreasing a numerical value of both or either one of the amplitude (r) and the frequency (h), and the vibration control unit 45 receives the information to suppress the vibration. In addition, in case that the vibration comes close to the natural frequency of the trough as a result of decreasing the frequency (h), no change of the frequency (h) is preferred. Thus, accumulation of the powder or granule can be suppressed and sufficient dispersion of the powder or granule is enabled.

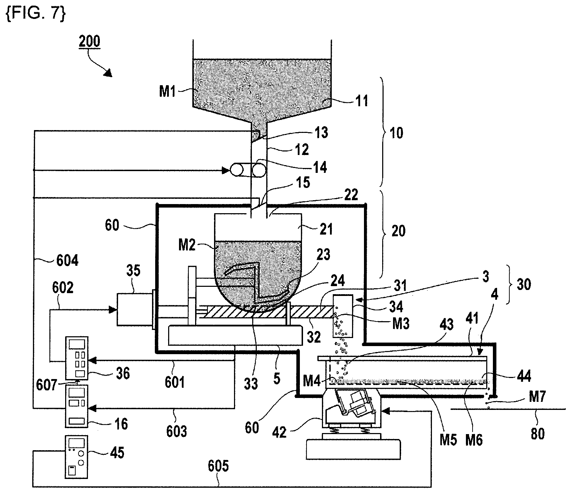

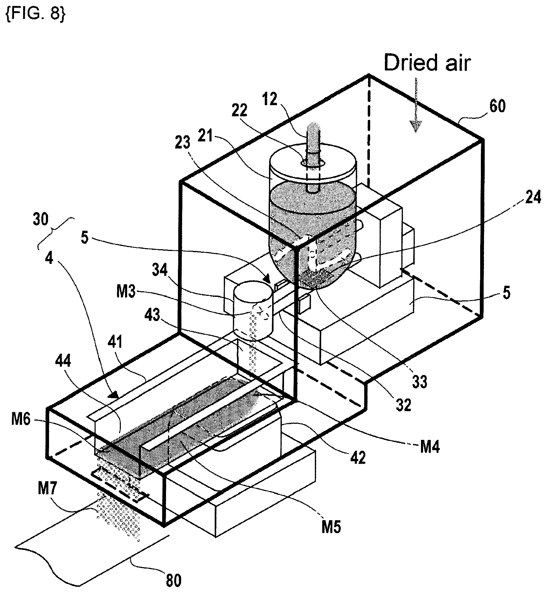

Next, an application device 200 as another preferred embodiment (second embodiment) according to the device for applying a powder or granule of the present invention, and a method for applying the powder or granule by the application device 200, are described with reference to FIGs. 7 and 8. FIG. 7 is a configuration diagram showing a device for applying the powder or granule according to a second embodiment. FIG. 8 is a partially enlarged perspective view showing an aspect of delivery of powder or granule from a screw feeder 3 to a vibrating conveyor unit 4 according to a second embodiment. In addition, an outside of the device is covered with a sealed vessel 60 in FIG. 7, but for understanding each step, the sealed vessel 60 becomes transparent and an outer shape of each member is shown using a solid line. In the embodiment, a member etc. identical with the member etc. in the first embodiment is shown by an identical symbol. Hereinafter, only points different from the first embodiment are described.

In the application device 200 for the powder or granule, the supply unit 20 and the conveyor unit 30 (the screw feeder 3 and the vibrating conveyor unit 4) of the above application device 100 for the powder or granule are arranged in the sealed vessel 60.

In the method for applying the powder or granule using this application device 200, steps identical with the steps shown in FIG. 4 are applied within the sealed vessel 60. Thus, contact of the powder or granule with humidity in outer air is suppressed while the powder or granule moves from the supply unit 20 to the sprinkling outlet 44 in the end of the conveyor unit 30, and therefore the powder or granule is hard to coagulate and does not adversely affect flowing properties. Further, accuracy of uniformization of the application amount is further improved. Moreover, in the method for manufacturing the heat generating element utilizing the method for applying the powder or granule within this sealed vessel, the heat generating element having excellent heating characteristics is further suitably obtained, which is preferred. On the occasion, dried air adjusted at a humidity of 30% or less and a temperature of 28°C or lower in the sealed vessel 60 is preferably blown. Moreover, the dried air further preferably has a humidity of 23% or less and a temperature of 23°C or lower and still further preferably has a humidity of 10% or less and a temperature of 23°C or lower.

If this dried air is configured, for example, to flow and be discharged from an upstream side to a downstream side of the application device 200, the humidity and the temperature in the sealed vessel as a whole can be efficiently kept constant. Thus, the application amount can be uniformized without being affected by a change of meteorological conditions such as temperature and humidity in a manufacturing place, which is preferred.

In the application device 200 according to the embodiment, as the sealed vessel 60, non-corrosive one or anti-corrosive one to the electrolyte powder or granule, such as nonmetal material including glass or stainless-steel material including SUS 316 is preferred, and sealability thereof means no intake of air in which humidity or temperature outside the sealed vessel 60 is not adjusted, and if this condition is satisfied, the sealed vessel 60 is not completely blocked from outside. Specific examples of the method for blowing dried air and a device therefor include a heatless air dryer and a membrane air dryer (dew point of outlet air at atmospheric pressure: 60°C).

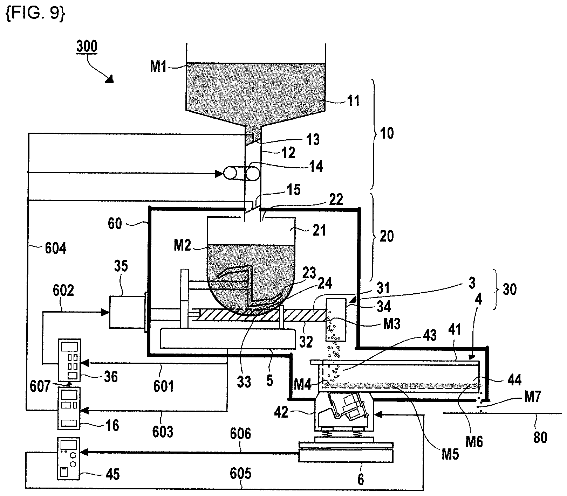

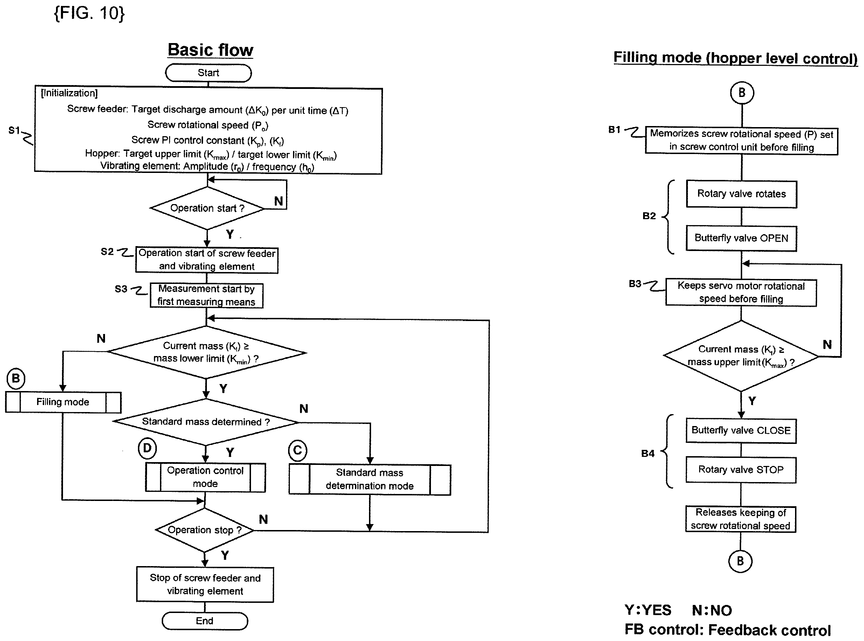

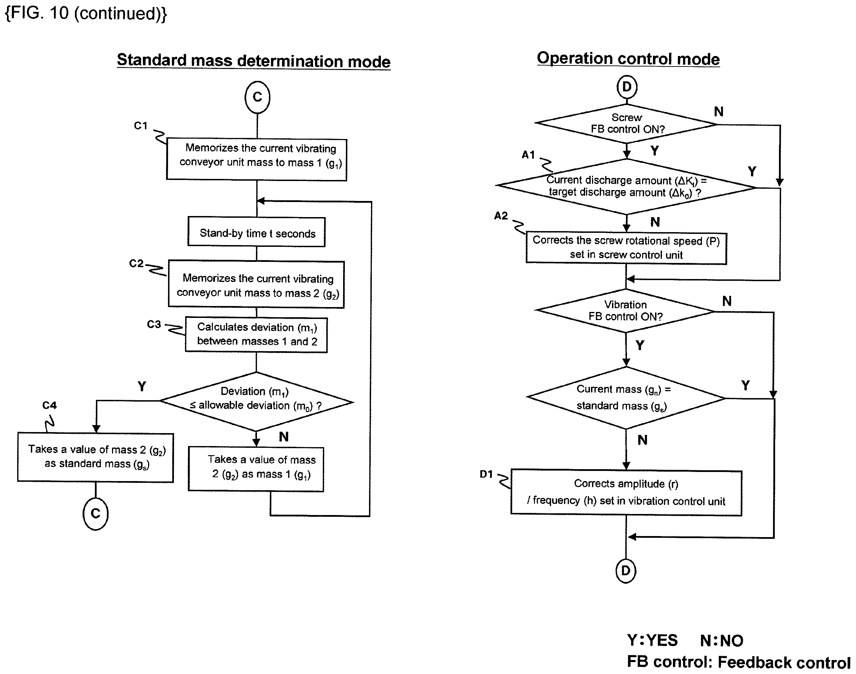

Next, an application device 300 and a method for applying a powder or granule by the application device 300, as another preferred embodiment (third embodiment) according to the device for applying a powder or granule of the present invention, are described referring to FIGs. 9 and 10. FIG. 9 is a configuration diagram showing a device for applying the powder or granule according to a third embodiment. FIG. 10 is a flowchart diagram showing a vibration control method in a vibrating conveyor unit 4 according to a third embodiment. In the embodiment, a member etc. identical with the member etc. in the first embodiment and the second embodiment is shown by an identical symbol. Hereinafter, only points different from the first embodiment and the second embodiment are described.

In an application device 300 for the powder or granule, a second measuring means 6 is installed in the vibrating conveyor unit 4 of the application device 200 for the powder or granule according to the above second embodiment. As the second measuring means 6, one similar to the above first measuring means 5 can be used. The second measuring means 6 continuously measures a mass of the vibrating conveyor unit 4 as a whole to send the measured value to the vibration control unit 45 as data (symbol 606 in FIG. 9). Based on this measured value, the vibration control unit 45 continuously calculates increase and decrease information of the powder or granule to perform feedback control for controlling the amplitude (r) and the frequency (h) of the vibrating element 42 to be suitable. Thus, the vibration control unit 45 can control the vibration of the vibrating element 42 to be in a relation of one on one between a discharge amount (q1) of the powder or granule M3 to the vibrating conveyor unit 4 from the screw feeder 3 and an application amount (q2) of the powder or granule M7 onto the conveyed base material 80 from the sprinkling outlet 44 of the vibrating conveyor unit 4. As a result, quantification of an application amount of the powder or granule is further suitably performed, which is preferred.

In the method for applying the powder or granule using this application device 300, steps shown in FIG. 10 are applied. In the steps shown in FIG. 10, a standard mass determining mode (C) and a correction control of amplitude (r) and frequency (h) of the vibrating element 42 in a second half of an operation control mode (D) are different from the steps shown in FIG. 4, and the points are described below.

In the standard mass determining mode (C), a mass to be a standard of judgment for correction of the amplitude (r) and the frequency (h) is calculated and determined. Firstly, when the steps S1 to S3 in the basic flow are carried out, measurement by the second measuring means 6 is started simultaneously with the feedback control in the vibration control unit 45. The second measuring means 6 measures a total mass of the vibrating conveyor unit 4 (the trough 41 and the vibrating element 42) and the powder or granule discharged thereon at a starting time point to memorize this mass as a mass 1 ($g_1$) in the vibration control unit 45 (symbol 606 in FIG. 9) (step C1). Next, after t seconds, the second measuring means 6 again measures a total mass of the vibrating conveyor unit 4 and the powder or granule discharged thereon to memorize this mass as a mass 2 ($g_2$) in the vibration control unit 45 (symbol 606 in FIG. 9) (step C2). The vibration control unit 45 calculates a deviation ($m_1$) from the mass 1 ($g_1$) and the mass 2 ($g_2$) based on the following Formula (3) (step C3). In addition, the above-mentioned powder or granule discharged on the vibrating conveyor unit 4 means the powder or granule remaining on the vibration conveyor unit 4 without sprinkled powder or granule.

$$(m_1) = g_2 - g_1 \quad \text{Formula (3)}$$

Further, the vibration control unit 45 compares a calculated deviation ($m_1$) with a preset allowable deviation ($m_0$), and in case of ($m_1 \leq m_0$), memorizes the mass 2 ($g_2$) as a standard mass ($g_s$) (step C4). On the other hand, in case of ($m_1 > m_0$), the vibration control unit 45 shifts the mass 2 ($g_2$) to a new mass 1 ($g_1$). Then, the second measuring means 6 again measures a mass of the vibrating conveyor unit 4 after t seconds. The vibration control unit 45 memorizes this measured value as a new mass 2 ($g_2$), and again calculates a deviation ($m_1$) to compare the calculated value with the preset allowable deviation ($m_0$). The vibration control unit 45 repeats the operation until an expression ($m_1 \leq m_0$) is satisfied, and at a time point of satisfying the expression ($m_1 \leq m_0$), memorizes a mass 2 ($g_2$) at that time as a standard mass ($g_s$).

This operation is shown in the following Formula (6) as described below. In addition, stand-by time means a period of measurement and is an arbitrary value. The allowable deviation means an allowable value of deflection of the measured values of the masses of the vibrating conveyor unit 4, which is caused by the vibration of the vibrating conveyor unit 4, and is an arbitrary value.

$$m_1 = (g_2 - g_1) \leq m_0 \quad \text{Formula (6)}$$

$g_1$ = mass of vibrating conveyor unit after t* (n-1) seconds

$g_2$ = mass of vibrating conveyor unit after t*n seconds

t = stand-by time (second)

n = number of times of measurement (n is an integer of 1 or more, and continuously increases one by one until satisfying an expression: $m_1 \leq m_0$.)

$m_0$ = allowable deviation

$m_1$ = calculated deviation

Next, in and after determination of the standard mass ($g_s$), in the operation control mode (D), the vibration control unit 45 performs feedback control of the amplitude (r) and the frequency (h) of the vibrating element 42 together with the feedback control (steps A1, A2) of the screw rotational speed ($P_0$) by the screw control unit 36. Specifically, a mass of the vibrating conveyor unit 4 (the trough 41 and the vibrating element 42) measured by the second measuring means 6 is first memorized as a current mass ($g_n$) in the vibration control unit 45. The vibration control unit 45 compares the current mass ($g_n$) with the standard mass ($g_s$), and in case where an expression: $g_n = g_s$ is not satisfied, performs correction control of the amplitude (r) and the frequency (h) of the vibrating element 42 based on a difference: $g_n - g_s$ (step D1). Specifically, a case where the difference: $g_n - g_s$ has a positive value is in a state in which the powder or granule M4 to the powder or granule M6 on the trough 41 have a mass larger than the standard mass ($g_s$), more specifically, an amount of the powder or granule M7 sprinkled from the sprinkling outlet 44 becomes smaller than an amount of the powder or granule M3 received from the screw feeder 3, and the powder or granule cannot be sufficiently sprinkled from the sprinkling outlet 44. Therefore, the vibration control unit 45 adjusts the amplitude (r) or the frequency (h) to increase the application amount. On the other hand, when the difference: $g_n - g_s$ has a negative value, the vibration control unit 45 adjusts the amplitude (r) or the frequency (h) to decrease the application amount.

In the application device 300 and the method for applying the powder or granule using the application device 300 according to the embodiment, accuracy of uniformization of the application amount is further improved by the feedback control in the vibrating conveyor unit 4. Then, in the method for manufacturing the heat generating element utilizing this method for applying the powder or granule, the heat generating element having excellent heating characteristics is further suitably obtained, which is preferred.

The device and method for applying the powder or granule according to the present invention are presented as ones suitably utilized to the method for manufacturing the heat generating element, but are limited thereto, and can be widely utilized without being limited by a kind of the powder or granule or an application target, for example, application of a super absorbent polymer to a sanitary material such as a diaper and a sanitary napkin etc., and application of a seasoning agent to snack food.

With regard to the above-mentioned embodiment, the present invention further discloses the method for applying the powder or granule, the device for applying the powder or granule and the method for manufacturing the heat generating element as described below.

<1> A method for applying a powder or granule on a base material to be continuously conveyed, wherein the powder or granule is continuously pulled out, by a screw feeder, from a supply unit for temporarily storing the powder or granule, the pulled-out powder or granule is allowed to fall to be received in a vibrating conveyor unit, the powder or granule is conveyed while being dispersed by vibration of a vibrating element of the vibrating conveyor unit, and the powder or granule is continuously applied on the base material from a sprinkling outlet of the vibrating conveyor unit.

<2> The method for applying the powder or granule according to the item <1>, wherein the powder or granule falls in line with a vertical line in which an outlet of the powder or granule in the screw feeder, a receiving position of the powder or granule in the vibrating conveyor unit as separated from the outlet and below the outlet, and the vibrating element are aligned, and the fallen powder or granule is conveyed in a horizontal direction from the receiving position to the sprinkling outlet of the other end while dispersing the powder or granule by vibration of the vibrating element.

<3> The method for applying the powder or granule according to the item <1 > or <2>, wherein a first measuring means continuously measures a total mass of the supply unit, the screw feeder and the powder or granule therein, and a screw control unit calculates a decrement of the powder or granule per time based on a plurality of the measured values, and controls a screw rotation speed of the screw feeder so that the calculated decrement coincides with a target discharge amount per time.

<4> The method for applying the powder or granule according to the item <3>, wherein the screw control unit compares a discharge amount ($\Delta K_t$) of the powder or granule per time (AT) to be calculated based on the measured value ($K_t$) received from the first measuring means, with a target discharge amount ($\Delta K_0$) of the powder or granule per time (AT) to give a correction instruction to increase a screw rotational speed in case of $\Delta K_t < \Delta K_0$, and to decrease the screw rotational speed in case of $\Delta K_t > \Delta K_0$, to a driving unit.

<5> The method for applying the powder or granule according to the item <3> or <4>, wherein, in case that the continuously measured value is lower than a target lower limit amount of a total mass obtained by combining a supply unit, a screw feeder and the powder or granule therein, a powder or granule supply amount control unit performs supplying control of the powder or granule in the supply unit up to the target upper limit amount.

<6> The method for applying the powder or granule according to any one of the items <1> to <5>, wherein a second measuring means continuously measures a total mass of the vibrating conveyor unit and the powder or granule discharged thereon, and a vibration control unit sets a mass ($g_2$) of the vibrating conveyor unit in case that a deviation ($m_1$) of the measured value calculated by Formula (6) becomes lower than an allowable deviation ($m_0$) as a standard mass ($g_s$), and the vibration control unit adjusts at least one of amplitude and frequency in the vibrating element, so that the mass ($g_n$) of the vibrating conveyor unit in coincides with the standard mass ($g_s$) in case that a measured mass ($g_n$) of the vibrating conveyor unit deviates from the standard mass ($g_s$).

$$m_1 = (g_2-g_1) \leq m_0 \qquad \text{Formula (6)}$$

$g_1$ = mass of vibrating conveyor unit after t* (n-1) seconds
$g_2$ = mass of vibrating conveyor unit after t*n seconds
t = stand-by time (second) (Stand-by time is an arbitrary value which is a period of the measurement.)
n = number of times of measurement (n is an integer of 1 or more, and continuously increases one by one until satisfying an expression: $m_1 \leq m_0$.)
$m_0$ = allowable deviation (Allowable deviation is an arbitrary value which is an allowable value of the deflection of the measured value of the mass of the vibrating conveyor unit caused by the vibration of the vibrating conveyor unit.)
$m_1$ = calculated deviation

<7> The method for applying the powder or granule according to any one of the items <1> to <6>, wherein a width dispersion state of the powder or granule is detected in the sprinkling outlet of the vibrating conveyor unit by a detecting means, and a vibration control unit adjusts at least any one of amplitude and frequency of the vibrating element based on the detected information.

<8> The method for applying the powder or granule according to any one of the items <1> to <7>, wherein the powder or granule has deliquescent or hygroscopic properties.

<9> The method for applying the powder or granule according to any one of the items <1> to <8>, comprising a step for slitting the base material on which the powder or granule is applied, into a plurality of rows in a width direction.

<10> The method for applying the powder or granule according to any one of the items <1> to <9>, wherein the powder or granule in the vibrating conveyor unit are branched into a plurality of rows at the sprinkling outlet to be continuously applied on the base material.

<11> The method for applying the powder or granule according to any one of the items <1> to <10>, wherein an average particle diameter of the powder or granule is 50 $\mu$m or more and 1,000 $\mu$m or less.

<12> A device for applying a powder or granule, comprising a supply unit of the powder or granule, and a conveyor unit for conveying and applying the powder or granule from the supply unit, wherein the conveyor unit has a screw feeder for pulling out the powder or granule from an inside of the supply unit, and a vibrating conveyor unit for conveying the pulled-out powder or granule while dispersing the powder or granule by vibration.

<13> The device for applying the powder or granule according to the item <12>,
wherein the screw feeder has an outlet for allowing the pulled-out powder or granule to fall downward, and the vibrating conveyor unit has a conveyor element for receiving powder or granule falling from the outlet to convey the powder or granule while dispersing the powder or granule, and a vibrating element for vibrating the conveyor element, and the conveyor element has a sprinkling outlet for applying the dispersed powder or granule; and
wherein the vibrating conveyor unit is separated from the outlet and positioned below the outlet, and a receiving position of the falling powder or granule in the conveyor element and the vibrating element are arranged in line with a vertical line with the outlet.

<14> The device for applying the powder or granule according to the item <13>, wherein, in a front view of the conveyor element along a direction perpendicular to the direction of conveying the powder or granule from the receiving position to the sprinkling outlet, a width central position of the conveyor element coincides with a width central position of the outlet.

<15> The device for applying the powder or granule according to any one the items <12> to <14>, comprising a first measuring means for continuously measuring a total mass of the supply unit, the screw feeder and the powder or granule therein; and a screw control unit for controlling a screw rotation speed of the screw feeder,
wherein the screw control unit calculates a decrement of the powder or granule per time in the screw feeder based on a plurality of measured values by the first measuring means, and controls the screw rotation speed of the screw feeder so as to allow the decrement per time to coincide with a target discharge amount per time.

<16> The device for applying the powder or granule according to the item <15>, comprising a stock unit disposed upstream of the supply unit and a powder or granule supply amount control unit,

Wherein the powder or granule supply amount control unit performs supplying control of the powder or granule from the stock unit to the supply unit up to a target upper limit amount of the total mass, in case that a measured value by the first measuring means is lower than a target lower limit amount of a total mass obtained by combining a supply unit, a screw feeder and the powder or granule therein.

<17> The device for applying the powder or granule according to any one of the items <12> to <16>, wherein the supply unit, the screw feeder and the vibrating conveyor unit are covered by a sealed vessel into which dried air adjusted for temperature and humidity is blow.

<18> The device for applying the powder or granule according to the item <17>, wherein the dried air is adjusted to have a humidity of 30% or less and a temperature of 28°C or lower.

<19> The device for applying the powder or granule according to any one of the items <12> to <18>, comprising a detecting means for detecting a traverse dispersion state of powder or granule in a sprinkling outlet of the vibrating conveyor unit; and a vibration control unit for adjusting at least any one of amplitude and frequency of the vibrating element based on the detected information.

<20> The device for applying the powder or granule according to any one of the items <12> to <19>, wherein the application device has a stock unit to be arranged upstream of the supply unit, and the stock unit has a stock tank, a distribution pipe of powder or granule, and a first butterfly valve, a rotary valve and a second butterfly valve in the distribution pipe.

<21> The device for applying the powder or granule according to any one of the items <12> to <20>, wherein the supply unit comprises a hopper having a storage body unit, and a distribution receiving opening of the accumulated powder or granule to the storage body unit, an agitator for agitating the storage powder or granule in the storage body unit, and a supply opening of the storage powder or granule.

<22> The device for applying the powder or granule according to any one of the items <13> to <21>, wherein a width of the conveyor element of the vibrating conveyor unit and a width of the sprinkling outlet of the conveyor element are formed to be wider than a width of the outlet of the screw feeder.

<23> The device for applying the powder or granule according to the item <16>, powder or granule from the stock unit to the supply unit is distributed by control of a rotary valve, a first butterfly valve and a second butterfly valve of the stock unit by the powder or granule supply amount control unit.

<24> The device for applying the powder or granule according to the item <19>,wherein a second measuring means for continuously measuring a mass of a vibrating conveyor unit to send the measured value to a vibration control unit is installed in a vibrating conveyor unit.

<25> The device for applying the powder or granule according to the item <15> or <24>, wherein, as the first measuring means or the second measuring means, a load-cell meter as an electric meter, an electromagnetic meter or a tuning fork meter is used.

<26> A method for manufacturing a heat generating element, wherein a step for applying a powder or granule of electrolyte, by using the method for applying the powder or granule according to any one of the items <1 > to <11>, on the base material to be continuously conveyed, and a step for coating a coating * composition containing no electrolyte and containing oxidizable metal particles and water are applied, in this order, in a reverse order or simultaneously.

<27> A method for manufacturing a heat generating element, wherein a step for applying a powder or granule of electrolyte, by using the device for applying the powder or granule according to any one of the items <12> to <25>, on the base material to be continuously conveyed, and a step for coating a coating composition containing no electrolyte and containing oxidizable metal particles and water are applied in this order, in a reverse order or simultaneously.

<28> The method for manufacturing the heat generating element according to the item <26> or <27>, wherein, as the electrolyte, sodium chloride, potassium chloride, calcium chloride, magnesium chloride, ferrous chloride or ferric chloride is used.

<29> The method for manufacturing the heat generating element according to any one of the items <26> to <28>, wherein an average particle diameter of the powder or granule is 50 $\mu$m or more and 1,000 $\mu$m or less.

<30> The method for manufacturing the heat generating element according to any one of the items <26> to <29>, wherein the base material is a fibrous sheet containing hydrophilic fibers or a fibrous sheet containing superabsorbent polymer particles.

EXAMPLE

The present invention will be described in more detail based on the following examples, but the invention is not intended to be limited thereto.

(Example 1)

A device having a basic constitution identical with the constitution of an application device 200 for the powder or granule as shown in FIG. 7 was used, and dried air was blown using a membrane air dryer, and thus an inside of a sealed vessel 60 was kept at a humidity of 20% or more and 21% or less, and a temperature of 23°C. In addition, an indoor environment outside the sealed vessel had a temperature of 23°C, and a humidity of 30 % or more and 70% or less. As a hopper, one having a maximum weight of 5 kg was used, and 3 kg of powder or granule was charged into the hopper. As a screw feeder 3, a twinscrew cassette weighing feeder (trade name), model number: CE-W-OD, manufactured by Kubota Corporation was used, a target discharge amount was set to 3.360 kg/h, and feedback control was set to OFF. As a vibrating conveyor unit 4, a vibrating feeder CF-1 (with a standard trough) manufactured by SINFONIA TECHNOLOGY CO., LTD. was used, and as a vibration control unit, a vibrating feeder controller C10-1VCF manufactured by the above identical company was used, and set to an amplitude of 45%, and a frequency of 70 Hz.

Under the above setup, a weight of electrolyte powder or granule M7 (sodium chloride, particle size: a degree of 250 $\mu$m or more and 400 $\mu$m or less) applied from a sprinkling outlet 44 of a trough 41 was continuously measured using a first measuring means (load-cell meter), the weight was divided by time per one sheet of heat generating element, and a fluctuation of an application amount corresponding to 300 sheets of heat generating elements was measured.

(Comparative Example 1)

A weight fluctuation of electrolyte powder or granule M3 discharged from an outlet 34 of a screw feeder 3 was measured after removing the vibrating conveyor unit 4 in Example 1. Other conditions were set up in a manner similar to Example 1.

(Comparative Example 2)

A weight fluctuation of electrolyte powder or granule M3 discharged from an outlet 34 of a screw feeder 3 was measured after removing the vibrating conveyor unit 4 in Example 1. Feedback control of a rotational speed of the screw feeder 3 was performed. In addition, PI control shown in Formula 2 was applied to the feedback control, and a PI control constant was set as described below.

proportional constant $K_P$ = 200
integration constant $K_I$ = 200

Therefore, when a deviation $\varepsilon$ arose, a rotational speed of the screw feeder 3 corresponding to 50% of the deviation was instantaneously corrected by a proportional operation to correct the rotational speed in an amount identical with the amount of the proportional operation for 20 seconds.

Other conditions were set up in a manner similar to Example 1.

From fluctuation data of the application amount corresponding to 300 sheets of heat generating elements in each of Example 1 and Comparative Examples 1 and 2, an average value, a maximum value, a minimum value and a standard deviation were calculated, respectively. Then, an index when the target discharge amount set to the screw feeder is taken as 1 is presented in Table 1 below.

{Table 1}

Table 1

|  | Ex 1 | C Ex 1 | C Ex 2 |
|---|---|---|---|
| Vibrating conveyor unit | Existence | Non-existence | Non-existence |
| Feedback control of screw feeder rotational speed | Non-existence | Non-existence | Existence |

(continued)

|  | | Ex 1 | C Ex 1 | C Ex 2 |
|---|---|---|---|---|
| Powder or granule application mass (target discharge amount is taken as 1) | Average value | 1.005 | 1.005 | 1.005 |
| | Maximum value | 1.055 | 2.132 | 1.111 |
| | Minimum value | 0.969 | 0.384 | 0.873 |
| | Standard deviation σ | 0.015 | 0.206 | 0.043 |
| "Ex" means Example according to this invention, and "C Ex" means Comparative Example. | | | | |

As is clear from the results presented in Table 1, in Example 1, a standard deviation σ is smaller, and accordingly application accuracy of the powder or granule is found to be further excellent in comparison with Comparative Example 1 and Comparative Example 2.

Having described our invention as related to this embodiments, it is our intention that the invention not be limited by any of the details of the description, unless otherwise specified, but rather be construed broadly within its spirit and scope as set out in the accompanying claims.

This nonprovisional application claims priority under 35 U.S.C. §119 (a) on Patent Application No. 2011-268413 filed in Japan on December 7, 2011, which is entirely herein incorporated by reference.

INDUSTRIAL APPLICABILITY

A method and a device for applying a powder or granule, and a method for manufacturing a heat generating element using the same according to the present invention produce an excellent effect of allowing uniformization of an application amount of the powder or granule applied on a base material to be continuously conveyed to control the application amount.

DESCRIPTION OF SYMBOLS

10 Stock unit
16 Powder or granule supply amount control unit
20 Supply unit
30 Conveyor unit
3 Screw feeder
31 Screw
32 Outer wall section
33 Intake
34 Outlet
35 Driving unit
36 Screw control unit
4 Vibrating conveyor unit
41 Trough
42 Vibrating element
43 Receiving position
44 Sprinkling outlet
45 Vibration control unit
5 First measuring means
6 Second measuring means
80 Base material (Base material sheet)
100,200,300 Application device for the powder or granule

**Claims**

1. A method for applying a powder or granule, which has deliquescent or hygroscopic properties, on a base material to be continuously conveyed,
   wherein the powder or granule is continuously pulled out, by a screw feeder (3), from a supply unit (20) for temporarily storing the powder or granule,
   the pulled-out powder or granule is allowed to fall to be received in a vibrating conveyor unit (4), the powder or granule is conveyed while being dispersed by vibration of a vibrating element (42) of the vibrating conveyor unit, and the powder or granule is continuously applied on the base material from a sprinkling outlet (44) of the vibrating conveyor unit;
   **characterized in that** a first measuring means (5) continuously measures the total mass of the supply unit, the screw feeder and the powder or granule therein, and a screw control unit (36) calculates the decrement of the powder or granule per time based on a plurality of the measured values,
   and controls the screw rotation speed of the screw feeder so that the calculated decrement coincides with a target discharge amount per time.

2. The method for applying the powder or granule according to Claim 1, wherein the powder or granule falls in line with a vertical line in which an outlet of the powder or granule in the screw feeder, a receiving position of the powder or granule in the vibrating conveyor unit as separated from the outlet and below the outlet, and the vibrating element are aligned, and the fallen powder or granule is conveyed in a horizontal direction from the receiving position to the sprinkling outlet of the other end while dispersing the powder or granule by vibration of the vibrating element.

3. The method for applying the powder or granule according to Claim 1 or 2, wherein, in case that the continuously measured value is lower than a target
   lower limit amount of a total mass obtained by combining a supply unit, a screw feeder and the powder or granule therein, a powder or granule supply amount control unit performs supplying control of the powder or granule in the supply unit up to a target upper limit amount.

4. The method for applying the powder or granule according to any one of Claims 1 to 3, wherein a second measuring means continuously measures a total mass of the vibrating conveyor unit and the powder or granule discharged thereon, and a vibration control unit sets a mass ($g_2$) of the vibrating conveyor unit in case that a deviation ($m_1$) of the measured value calculated by Formula (6) becomes lower than an allowable deviation ($m_0$) as a standard mass ($g_s$), and the vibration control unit adjusts at least one of amplitude and frequency in the vibrating element, so that the mass ($g_n$) of the vibrating conveyor unit coincides with the standard mass ($g_s$) in case that a measured mass ($g_n$) of the vibrating conveyor unit deviates from the standard mass ($g_s$).

$$m_1 = (g_2 - g_1) \leq m_0 \qquad \text{Formula (6)}$$

$g_1$ = mass of vibrating conveyor unit after t* (n-1) seconds
$g_2$ = mass of vibrating conveyor unit after t*n seconds
t = stand-by time (second) (Stand-by time is an arbitrary value which is a period of the measurement.)
n = number of times of measurement (n is an integer of 1 or more, and continuously increases one by one until satisfying an expression: $m_1 \leq m_0$.)
$m_0$ = allowable deviation (Allowable deviation is an arbitrary value which is an allowable value of the deflection of the measured value of the mass of the vibrating conveyor unit caused by the vibration of the vibrating conveyor unit.)
$m_1$ = calculated deviation

5. The method for applying the powder or granule according to any one of Claims 1 to 4, wherein a width dispersion state of the powder or granule is detected in the sprinkling outlet of the vibrating conveyor unit by a detecting means, and a vibration control unit adjusts at least any one of amplitude and frequency of the vibrating element based on the detected information.

6. A device for applying a powder or granule, which has deliquescent or hygroscopic properties, comprising a supply unit (20) of the powder or granule, and a conveyor unit (30) for conveying and applying the powder or granule from the supply unit (20), wherein the conveyor unit (30) has a screw feeder (3) for pulling out the powder or granule from an inside of the supply unit, and a vibrating conveyor unit (4) for conveying the pulled-out powder or granule while dispersing the powder or granule by vibration,

**characterized by** comprising a first measuring means (5) for continuously measuring the total mass of the supply unit, the screw feeder and the powder or granule therein; and a screw control unit (36) for controlling the screw rotation speed of the screw feeder (3),

wherein the screw control unit (36) calculates the decrement of the powder or granule per time in the screw feeder based on a plurality of measured values by the first measuring means (5), and controls the screw rotation speed of the screw feeder (3) so as to allow the decrement per time to coincide with a target discharge amount per time.

7. The device for applying the powder or granule according to Claim 6,

wherein the screw feeder (3) has an outlet (34) for allowing the pulled-out powder or granule to fall downward, and the vibrating conveyor unit (4) has a conveyor element for receiving the powder or granule falling from the outlet (34) to convey the powder or granule while dispersing the powder or granule, and a vibrating element (42) for vibrating the conveyor element, and the conveyor element has a sprinkling outlet (44) for applying the dispersed powder or granule; and

wherein the vibrating conveyor unit (4) is separated from the outlet (34) and positioned below the outlet (34), and a receiving position of the falling powder or granule in the conveyor element and the vibrating element are arranged in line with a vertical line with the outlet (34).

8. The device for applying the powder or granule according to Claim 7, wherein, in a front view of the conveyor element along a direction perpendicular to the direction of conveying the powder or granule from the receiving position to the sprinkling outlet, a width central position of the conveyor element coincides with a width central position of the outlet (34).

9. The device for applying the powder or granule according to any one of Claims 6 to 8, comprising a stock unit (10) disposed upstream of the supply unit and a powder or granule supply amount control unit (16),

wherein the powder or granule supply amount control unit (16) performs supplying control of the powder or granule from the stock unit (10) to the supply unit up to a target upper limit amount of the total mass, in case that a measured value by the first measuring means (5) is lower than a target lower limit amount of a total mass obtained by combining a supply unit (20), a screw feeder (3) and the powder or granule therein.

10. The device for applying the powder or granule according to any one of Claims 6 to 9, wherein the supply unit (20), the screw feeder (3) and the vibrating conveyor unit (4) are covered by a sealed vessel into which dried air adjusted for temperature and humidity is blown.

11. The device for applying the powder or granule according to any one of Claims 6 to 10, comprising a detecting means for detecting a traverse dispersion state of the powder or granule in a sprinkling outlet of the vibrating conveyor unit (4); and a vibration control unit (45) for adjusting at least any one of amplitude and frequency of the vibrating element based on the detected information.

12. A method for manufacturing a heat generating element, comprising the method for applying a powder or granule according to any one of Claims 1 to 5, and further comprising a step for coating a coating composition containing no electrolyte and containing oxidizable metal particles and water are applied in this order, in a reverse order or simultaneously.

**Patentansprüche**

1. Verfahren zum Auftragen eines Pulvers oder Granulats, das zerfließliche oder hygroskopische Eigenschaften hat, auf ein kontinuierlich zu förderndes Grundmaterial, wobei das Pulver oder Granulat durch einen Schneckenaufgeber (3) aus einer Zufuhreinheit (20) zum zeitweiligen Speichern des Pulvers oder Granulats kontinuierlich abgezogen wird, das abgezogene Pulver oder Granulat herabfallen gelassen wird, um in einer Schwingfördereinheit (4) aufgenommen zu werden, das Pulver oder Granulat gefördert wird, während es durch Schwingung eines Schwingelements (42) der Schwingfördereinheit verteilt wird, und das Pulver oder Granulat aus einem Berieselungsauslass (44) der Schwingfördereinheit auf das Grundmaterial kontinuierlich aufgetragen wird;

**dadurch gekennzeichnet, dass** eine erste Messeinrichtung (5) die Gesamtmasse der Zufuhreinheit, des Schneckenaufgebers und des Pulvers oder Granulats darin kontinuierlich misst und eine Schneckensteuereinheit (36) die zeitliche Abnahme des Pulvers oder Granulats auf der Grundlage mehrerer der Messwerte berechnet und die Schneckendrehzahl des Schneckenaufgebers so steuert, dass die berechnete Abnahme mit einer zeitlichen Soll-Abgabemenge zusammenfällt.

2. Verfahren zum Auftragen des Pulvers oder Granulats nach Anspruch 1, wobei das Pulver oder Granulat in Übereinstimmung mit einer Vertikalen herabfällt, auf der ein Auslass des Pulvers oder Granulats im Schneckenaufgeber, eine Aufnahmeposition des Pulvers oder Granulats in der Schwingfördereinheit getrennt vom Auslass und unter dem Auslass sowie das Schwingelement ausgerichtet sind, und das herabgefallene Pulver oder Granulat in horizontaler Richtung von der Aufnahmeposition zum Berieselungsauslass des anderen Endes gefördert wird, während das Pulver oder Granulat durch Schwingung des Schwingelements verteilt wird.

3. Verfahren zum Auftragen des Pulvers oder Granulats nach Anspruch 1 oder 2, wobei in dem Fall, dass der kontinuierlich gemessene Wert niedriger als eine untere Soll-Grenzmenge einer Gesamtmasse ist, die durch Kombinieren einer Zufuhreinheit, eines Schneckenaufgebers und des Pulvers oder Granulats darin erhalten wird, eine Pulver- oder Granulatzufuhrmengen-Steuereinheit eine Zufuhrsteuerung des Pulvers oder Granulats in der Zufuhreinheit bis zu einer oberen Soll-Grenzmenge durchführt.

4. Verfahren zum Auftragen des Pulvers oder Granulats nach einem der Ansprüche 1 bis 3, wobei eine zweite Messeinrichtung eine Gesamtmasse der Schwingfördereinheit und des darauf abgegebenen Pulvers oder Granulats kontinuierlich misst und eine Schwingungssteuereinheit eine Masse ($g_2$) der Schwingfördereinheit in dem Fall, dass eine Abweichung ($m_1$) des durch Formel (6) berechneten Messwerts niedriger als eine zulässige Abweichung ($m_0$) wird, als Standardmasse ($g_s$) festlegt und die Schwingungssteuereinheit die Amplitude und/oder Frequenz im Schwingelement einstellt, so dass die Masse ($g_n$) der Schwingfördereinheit mit der Standardmasse ($g_s$) in dem Fall zusammenfällt, dass eine gemessene Masse ($g_n$) der Schwingfördereinheit von der Standardmasse ($g_s$) abweicht.

$$m_1 = (g_2 - g_1) \leq m_0 \qquad\qquad \text{Formel (6)}$$

$g_1$ = Masse der Schwingfördereinheit nach t*(n-1) Sekunden
$g_2$ = Masse der Schwingfördereinheit nach t*n Sekunden
t = Bereitschaftszeit (Sekunde) (die Bereitschaftszeit ist ein beliebiger Wert, der eine Periode der Messung ist)
n = Häufigkeit der Messung (n ist eine ganze Zahl von mindestens 1 und steigt kontinuierlich um eins, bis ein Ausdruck $m_1 \leq m_0$ erfüllt ist)
$m_0$ = zulässige Abweichung (die zulässige Abweichung ist ein beliebiger Wert, der ein zulässiger Wert der Ablenkung des Messwerts der Masse der Schwingfördereinheit, hervorgerufen durch die Schwingung der Schwingfördereinheit, ist)
$m_1$ = berechnete Abweichung

5. Verfahren zum Auftragen des Pulvers oder Granulats nach einem der Ansprüche 1 bis 4, wobei ein Breitenverteilungszustand des Pulvers oder Granulats im Berieselungsauslass der Schwingfördereinheit durch eine Detektionseinrichtung detektiert wird und eine Schwingungssteuereinheit die Amplitude und/oder Frequenz des Schwingelements auf der Grundlage der detektierten Informationen einstellt.

6. Vorrichtung zum Auftragen eines Pulvers oder Granulats, das zerfließliche oder hygroskopische Eigenschaften hat, mit einer Zufuhreinheit (20) des Pulvers oder Granulats und einer Fördereinheit (30) zum Fördern und Auftragen des Pulvers oder Granulats von der Zufuhreinheit (20), wobei die Fördereinheit (30) einen Schneckenaufgeber (3) zum Abziehen des Pulvers oder Granulats aus einem Innenraum der Zufuhreinheit und eine Schwingfördereinheit (4) zum Fördern des abgezogenen Pulvers oder Granulats hat, während das Pulver oder Granulat durch Schwingung verteilt wird,
**dadurch gekennzeichnet, dass** sie aufweist: eine erste Messeinrichtung (5) zum kontinuierlichen Messen der Gesamtmasse der Zufuhreinheit, des Schneckenaufgebers und des Pulvers oder Granulats darin; und eine Schneckensteuereinheit (36) zum Steuern der Schneckendrehzahl des Schneckenaufgebers (3), wobei die Schneckensteuereinheit (36) die zeitliche Abnahme des Pulvers oder Granulats im Schneckenaufgeber auf der Grundlage mehrerer Messwerte durch die erste Messeinrichtung (5) berechnet und die Schneckendrehzahl des Schneckenaufgebers (3) so steuert, dass die zeitliche Abnahme mit einer zeitlichen Soll-Abgabemenge zusammenfallen kann.

7. Vorrichtung zum Auftragen des Pulvers oder Granulats nach Anspruch 6,
wobei der Schneckenaufgeber (3) einen Auslass (34) zum Herabfallenlassen des abgezogenen Pulvers oder Granulats hat und die Schwingfördereinheit (4) ein Förderelement zum Aufnehmen des vom Auslass (34) herabfallenden Pulvers oder Granulats, um das Pulver oder Granulat zu fördern, während das Pulver oder Granulat verteilt wird, sowie ein Schwingelement (42) zum Schwingen des Förderelements hat und das Förderelement einen Berieselungsauslass (44) zum Auftragen des verteilten Pulvers oder Granulats hat; und

wobei die Schwingfördereinheit (4) vom Auslass (34) getrennt und unter dem Auslass (34) positioniert ist und eine Aufnahmeposition des herabfallenden Pulvers oder Granulats im Förderelement und Schwingelement in Übereinstimmung mit einer Vertikalen mit dem Auslass (34) angeordnet sind.

8. Vorrichtung zum Auftragen des Pulvers oder Granulats nach Anspruch 7, wobei in Vorderansicht des Förderelements entlang einer senkrechten Richtung zur Förderrichtung des Pulvers oder Granulats von der Aufnahmeposition zum Berieselungsauslass eine Breitenmittelposition des Förderelements mit einer Breitenmittelposition des Auslasses (34) zusammenfällt.

9. Vorrichtung zum Auftragen des Pulvers oder Granulats nach einem der Ansprüche 6 bis 8 mit einer Bestandseinheit (10), die stromaufwärts von der Zufuhreinheit angeordnet ist, und einer Pulver- oder Granulatzufuhrmengen-Steuereinheit (16), wobei die Pulver- oder Granulatzufuhrmengen-Steuereinheit (16) eine Zufuhrsteuerung des Pulvers oder Granulats aus der Bestandseinheit (10) zur Zufuhreinheit bis zu einer oberen Soll-Grenzmenge der Gesamtmasse in dem Fall durchführt, dass ein Messwert durch die erste Messeinrichtung (5) niedriger als eine untere Soll-Grenzmenge einer Gesamtmasse ist, die durch Kombinieren einer Zufuhreinheit (20), eines Schneckenaufgebers (3) und des Pulvers oder Granulats darin erhalten wird.

10. Vorrichtung zum Auftragen des Pulvers oder Granulats nach einem der Ansprüche 6 bis 9, wobei die Zufuhreinheit (20), der Schneckenaufgeber (3) und die Schwingfördereinheit (4) durch einen abgedichteten Behälter abgedeckt sind, in den temperatur- und feuchtigkeitseingestellte getrocknete Luft eingeblasen wird.

11. Vorrichtung zum Auftragen des Pulvers oder Granulats nach einem der Ansprüche 6 bis 10 mit einer Detektionseinrichtung zum Detektieren eines Querverteilungszustands des Pulvers oder Granulats in einem Berieselungsauslass der Schwingfördereinheit (4); und einer Schwingungssteuereinheit (45) zum Einstellen der Amplitude und/oder Frequenz des Schwingelements auf der Grundlage der detektierten Informationen.

12. Verfahren zur Herstellung eines Wärmeerzeugungselements, das das Verfahren zum Auftragen eines Pulvers oder Granulats nach einem der Ansprüche 1 bis 5 aufweist und ferner aufweist: einen Schritt zum Beschichten mit einer Beschichtungszusammensetzung, die keinen Elektrolyt enthält und oxidierbare Metallpartikel und Wasser enthält, die in dieser Reihenfolge, in umgekehrter Reihenfolge oder gleichzeitig aufgetragen werden.

## Revendications

1. Procédé pour appliquer une poudre ou granule, qui présente des propriétés déliquescentes ou hygroscopiques, sur un matériau de base à acheminer en continu, dans lequel la poudre ou granule est retirée en continu, par un distributeur à vis (3), d'une unité d'alimentation (20) pour stocker temporairement la poudre ou granule, la poudre ou granule retirée est autorisée à tomber pour être reçue dans une unité de transport vibrante (4), la poudre ou granule est transportée tout en étant dispersée par vibration d'un élément vibrant (42) de l'unité de transport vibrante, et la poudre ou granule est appliquée en continu sur le matériau de base depuis une sortie de pulvérisation (44) de l'unité de transport vibrante ;
   **caractérisé en ce qu'**un premier moyen de mesure (5) mesure en continu la masse totale de l'unité d'alimentation, du distributeur à vis et de la poudre ou granule à l'intérieur, et une unité de commande de vis (36) calcule le décrément de la poudre ou granule au fil du temps sur la base d'une pluralité de valeurs mesurées, et commande la vitesse de rotation de vis du distributeur à vis de telle sorte que le décrément calculé coïncide avec une quantité de décharge cible par temps.

2. Procédé pour appliquer la poudre ou granule selon la revendication 1, dans lequel la poudre ou granule tombe alignée sur une ligne verticale dans laquelle une sortie de la poudre ou granule dans le distributeur à vis, une position de réception de la poudre ou granule dans l'unité de transport vibrante séparée de la sortie et sous la sortie, et l'élément vibrant sont alignés, et la poudre ou granule tombée est transportée dans une direction horizontale depuis la position de réception jusqu'à la sortie de pulvérisation de l'autre extrémité tout en dispersant la poudre ou granule par vibration de l'élément vibrant.

**3.** Procédé pour appliquer la poudre ou granule selon la revendication 1 ou 2, dans lequel, dans le cas où la valeur mesurée en continu est inférieure à une quantité limite inférieure cible d'une masse totale obtenue en combinant une unité d'alimentation, un distributeur à vis et la poudre ou granule contenue, une unité de commande de quantité d'alimentation en poudre ou granule effectue une commande d'alimentation de la poudre ou granule dans l'unité d'alimentation jusqu'à une quantité limite supérieure cible.

**4.** Procédé pour appliquer la poudre ou granule selon l'une quelconque des revendications 1 à 3, dans lequel un second moyen de mesure mesure en continu une masse totale de l'unité de transport vibrante et de la poudre ou granule déchargée sur celle-ci, et une unité de commande de vibration règle une masse ($g_2$) de l'unité de transport vibrante dans le cas où un écart ($m_1$) de la valeur mesurée calculée par la Formule (6) devient inférieur à un écart admissible ($m_0$) comme masse standard ($g_s$), et l'unité de commande de vibration ajuste au moins une grandeur parmi l'amplitude et la fréquence dans l'élément vibrant, de telle sorte que la masse ($g_n$) de l'unité de transport vibrante coïncide avec la masse standard ($g_s$) dans le cas où une masse mesurée ($g_n$) de l'unité de transport vibrante dévie de la masse standard ($g_s$).

$$m_1 = (g_2 - g_1) \leq m_0 \qquad \text{Formule (6)}$$

$g_1$ = masse de l'unité de transport vibrante après $t*(n-1)$ secondes
$g_2$ = masse de l'unité de transport vibrante après $t*n$ secondes
t = temps d'attente (seconde) (Le temps d'attente est une valeur arbitraire qui est une période de la mesure)
n = nombre d'occurrences de mesure (n est un entier de 1 ou plus, et augmente en continu d'un en un jusqu'à satisfaire une expression : $m_1 \leq m_0$)
$m_0$ = écart admissible (L'écart admissible est une valeur arbitraire qui est une valeur admissible de la déviation de la valeur mesurée de la masse de l'unité de transport vibrante provoquée par la vibration de l'unité de transport vibrante)
$m_1$ = écart calculé

**5.** Procédé pour appliquer la poudre ou granule selon l'une quelconque des revendications 1 à 4, dans lequel un état de dispersion en largeur de la poudre ou granule est détecté dans la sortie de pulvérisation de l'unité de transport vibrante par un moyen de détection, et une unité de commande de vibration ajuste au moins une grandeur parmi l'amplitude et la fréquence de l'élément vibrant sur la base des informations détectées.

**6.** Dispositif pour appliquer une poudre ou granule, qui présente des propriétés déliquescentes ou hygroscopiques, comprenant une unité d'alimentation (20) de la poudre ou granule, et une unité de transport (30) pour transporter et appliquer la poudre ou granule provenant de l'unité d'alimentation (20), dans lequel l'unité de transport (30) possède un distributeur à vis (3) pour retirer la poudre ou granule d'un intérieur de l'unité d'alimentation, et une unité de transport vibrante (4) pour transporter la poudre ou granule retirée tout en dispersant la poudre ou granule par vibration,
**caractérisé en ce qu'**il comprend un premier moyen de mesure (5) pour mesurer en continu la masse totale de l'unité d'alimentation, du distributeur à vis et de la poudre ou granule à l'intérieur ; et une unité de commande de vis (36) pour commander la vitesse de rotation de vis du distributeur à vis (3),
dans lequel l'unité de commande de vis (36) calcule le décrément de la poudre ou granule au fil du temps dans le distributeur à vis sur la base d'une pluralité de valeurs mesurées par le premier moyen de mesure (5), et commande la vitesse de rotation de vis du distributeur à vis (3) de manière à faire coïncider le décrément au fil du temps avec une quantité de décharge cible au fil du temps.

**7.** Dispositif pour appliquer la poudre ou granule selon la revendication 6,
dans lequel le distributeur à vis (3) comporte une sortie (34) pour permettre à la poudre ou granule retirée de tomber, et l'unité de transport vibrante (4) possède un élément de transport pour recevoir la poudre ou granule tombant de la sortie (34) afin de transporter la poudre ou granule tout en dispersant la poudre ou granule, et un élément vibrant (42) pour faire vibrer l'élément de transport, et l'élément de transport comporte une sortie de pulvérisation (44) pour appliquer la poudre ou granule dispersée ; et
dans lequel l'unité de transport vibrante (4) est séparée de la sortie (34) et positionnée sous la sortie (34), et une position de réception de la poudre ou granule tombant dans l'élément de transport et l'élément vibrant sont agencés alignés sur une ligne verticale avec la sortie (34).

**8.** Dispositif pour appliquer la poudre ou granule selon la revendication 7, dans lequel, sur une vue de face de l'élément de transport le long d'une direction perpendiculaire à la direction de transport de la poudre ou granule depuis la position de réception vers la sortie de pulvérisation, une position centrale en largeur de l'élément de transport coïncide avec une position centrale en largeur de la sortie (34).

**9.** Dispositif pour appliquer la poudre ou granule selon l'une quelconque des revendications 6 à 8, comprenant une unité de stock (10) disposée en amont de l'unité d'alimentation et une unité de commande de quantité d'alimentation en poudre ou granule (16),
dans lequel l'unité de commande de quantité d'alimentation en poudre ou granule (16) effectue une commande d'alimentation de la poudre ou granule depuis l'unité de stock (10) vers l'unité d'alimentation jusqu'à une quantité limite supérieure cible de la masse totale, dans le cas où une valeur mesurée par le premier moyen de mesure (5) est inférieure à une quantité limite inférieure cible d'une masse totale obtenue en combinant une unité d'alimentation (20), un distributeur à vis (3) et la poudre ou granule contenue.

**10.** Dispositif pour appliquer la poudre ou granule selon l'une quelconque des revendications 6 à 9, dans lequel l'unité d'alimentation (20), le distributeur à vis (3) et l'unité de transport vibrante (4) sont recouverts d'une cuve étanche dans laquelle de l'air séché ajusté en température et humidité est soufflé.

**11.** Dispositif pour appliquer la poudre ou granule selon l'une quelconque des revendications 6 à 10, comprenant un moyen de détection pour détecter un état de dispersion de traversée de la poudre ou granule dans une sortie de pulvérisation de l'unité de transport vibrante (4) ; et une unité de commande de vibration (45) pour ajuster au moins une grandeur parmi l'amplitude et la fréquence de l'élément vibrant sur la base des informations détectées.

**12.** Procédé pour fabriquer un élément de génération de chaleur, comprenant le procédé pour appliquer une poudre ou granule selon l'une quelconque des revendications 1 à 5, et comprenant en outre une étape d'étalage d'une composition de revêtement ne contenant pas d'électrolyte et contenant des particules métalliques oxydables et de l'eau, appliqués dans cet ordre, dans un ordre inverse ou simultanément.

EP 2 789 400 B1

{FIG. 1}

{FIG. 2}

(a)

(b-1)

(b-2)

{FIG. 3}

{FIG. 4}

### Basic flow

Start

S1 — [Initialization]
Screw feeder: Target discharge amount ($\Delta K_0$) per unit time ($\Delta T$)
Screw rotational speed ($P_0$)
Screw PI control constant ($K_p$), ($K_i$)
Hopper: Target upper limit ($K_{max}$) / target lower limit ($K_{min}$)
Vibrating element: Amplitude ($r_0$) / frequency ($h_0$)

Operation start ? — N

Y

S2 — Operation start of screw feeder and vibrating element

S3 — Measurement start by first measuring means

Current mass ($K_t$) ≥ mass upper limit ($K_{min}$) ? — N

Y

(B) Filling mode

(A) Operation control mode

Operation stop ? — N

Y

Stop of screw feeder and vibrating element

End

### Operation control mode

(A)

Screw FB control ON? — N

Y

A1 — Current discharge amount ($\Delta K_t$) = target discharge amount ($\Delta k_0$) ? — Y

N

A2 — Corrects the screw rotational speed (P) set in screw control unit

(A)

### Filling mode (hopper level control)

(B)

B1 — Memorizes screw rotational speed (P) set in screw control unit before filling

B2 — Rotary valve rotates

Butterfly valve OPEN

B3 — Keeps servo motor rotational speed before filling

Current mass ($K_t$) ≥ mass upper limit ($K_{max}$) ? — N

Y

B4 — Butterfly valve CLOSE

Rotary valve STOP

Releases keeping of screw rotational speed

(B)

Y:YES   N:NO
FB control: Feedback control

{FIG. 5}

{FIG. 6}

{FIG. 7}

{FIG. 8}

Dried air

{FIG. 9}

{FIG. 10}

## Basic flow

```
( Start )

[Initialization]
    Screw feeder: Target discharge amount (ΔK₀) per unit time (ΔT)
                  Screw rotational speed (P₀)
                  Screw PI control constant (Kₚ), (Kᵢ)
    Hopper: Target upper limit (Kₘₐₓ) / target lower limit (Kₘᵢₙ)
    Vibrating element: Amplitude (r₀) / frequency (h₀)
```
S1

Operation start ?  — N

Y

S2 — Operation start of screw feeder and vibrating element

S3 — Measurement start by first measuring means

Current mass ($K_I$) ≥ mass lower limit ($K_{min}$) ?  — N

Y

(B) Filling mode

Standard mass determined ?  — N

(D)  Y

Operation control mode

(C) Standard mass determination mode

Operation stop ?  — N

Y

Stop of screw feeder and vibrating element

( End )

## Filling mode (hopper level control)

(B)

B1 — Memorizes screw rotational speed (P) set in screw control unit before filling

B2 {
Rotary valve rotates

Butterfly valve OPEN
}

B3 — Keeps servo motor rotational speed before filling

Current mass ($K_I$) ≥ mass upper limit ($K_{max}$) ?  — N

Y

B4 {
Butterfly valve CLOSE

Rotary valve STOP
}

Releases keeping of screw rotational speed

(B)

Y:YES    N:NO
FB control: Feedback control

EP 2 789 400 B1

{FIG. 10 (continued)}

## Standard mass determination mode

(C)

C1 — Memorizes the current vibrating conveyor unit mass to mass 1 ($g_1$)

Stand-by time t seconds

C2 — Memorizes the current vibrating conveyor unit mass to mass 2 ($g_2$)

C3 — Calculates deviation ($m_1$) between masses 1 and 2

Deviation ($m_1$) ≤ allowable deviation ($m_0$) ?

Y → C4 — Takes a value of mass 2 ($g_2$) as standard mass ($g_s$)

N → Takes a value of mass 2 ($g_2$) as mass 1 ($g_1$)

(C)

## Operation control mode

(D)

Screw FB control ON? — N

Y

A1 — Current discharge amount ($\Delta K_i$) = target discharge amount ($\Delta k_0$) ? — Y

N

A2 — Corrects the screw rotational speed (P) set in screw control unit

Vibration FB control ON? — N

Y

Current mass ($g_n$) = standard mass ($g_s$) — Y

N

D1 — Corrects amplitude (r) / frequency (h) set in vibration control unit

(D)

Y:YES    N:NO
FB control: Feedback control

**EP 2 789 400 B1**

## REFERENCES CITED IN THE DESCRIPTION

**Patent documents cited in the description**

- JP 4341368 A
- JP 11153473 A
- JP H1099367 A